# EUROPEAN PATENT APPLICATION

(11) **EP 4 023 295 A1**
(43) Date of publication of application: **06.07.2022**
(21) Application number: 20383169.8
(22) Date of filing: 29.12.2020
(51) Int. Cl.: A61P 35/00, C07K 7/56, C07K 14/47

(54) **VEGF-REGULATING PEPTIDES**

(71) Applicant: IDP Discovery Pharma, S.L., 08028 Barcelona (ES)
(72) Inventor: ESTEBAN MARTÍN, Santiago, 08028 BARCELONA (ES); NEVOLA, Laura, 08028 BARCELONA (ES); MORENO RAJA, Miguel Ramon, 03204 ELCHE (ES); MONTENEGRO ARCE, María Fernanda, 36208 VIGO (ES); RODRÍGUEZ LÓPEZ, José Neptuno, 30009 MURCIA (ES)
(74) Representative: ZBM Patents - Zea, Barlocci & Markvardsen

(57) **Abstract**

The invention provides a peptide or a pharmaceutical salt thereof having a length from 14 to 50 and comprising a sequence having at least a 85% of sequence identity with respect to SEQ ID NO: 1, wherein "m", "n", "p", and "q" represent integers and are selected from 0 and 1; the C-terminal end corresponds to -C(O)R₄; and N-terminal end corresponds to -NHR₅; the peptide optionally comprising a linker biradical "L" of formula (I) connecting an alpha carbon atom of an amino acid located at position "i" with an alpha carbon atom of an amino acid located at position "i+4" or "i+7" in the peptide sequence SEQ ID NO: 1.

The present invention also provides fusion proteins and pharmaceutical compositions comprising the peptides of the invention as well as their use in therapy and, particularly, in the treatment of a disease caused by an increase or reduction in VEGF levels.

SEQUENCE LISTING

<110>  IDP DISCOVERY PHARMA S.L.

<120>  VEGF_regulating peptides

<130>  P5667EP00

<160>  35

<170>  PatentIn version 3.5

<210>  1
<211>  18
<212>  PRT
<213>  Artificial Sequence

<220>
<223>  peptide



<220>
<221>  MISC_FEATURE
<222>  (1)..(1)
<223>  X represents (Leu)m, wherein m is 0 or 1

<220>
<221>  MISC_FEATURE
<222>  (1)..(1)
<223>  N-terminal end corresponds to -NHR5, wherein R5 is selected from
       the group consisting of -H and (C1-C20)alkyl

<220>
<221>  MISC_FEATURE
<222>  (2)..(2)
<223>  X represents (Asp)n, wherein n is 0 or 1

<220>
<221>  MISC_FEATURE
<222>  (17)..(17)
<223>  X represents (Arg)p, wherein p is 0 or 1

<220>
<221>  MISC_FEATURE
<222>  (18)..(18)
<223>  X represents (Lys)q, wherein q is 0 or 1

<220>
<221>  MISC_FEATURE
<222>  (18)..(18)
<223>  C-terminal end corresponds to -C(O)R4, wherein R4 is a radical
       selected from the group consisting of -OH and -NR17R18;

<400>  1

Xaa Xaa Lys Ala Ser Val Met Arg Leu Thr Ile Ser Tyr Leu Arg Val
1               5                   10                  15


Xaa Xaa

<210>  2
<211>  18
<212>  PRT
<213>  Artificial Sequence

<220>
<223>  peptide



<220>
<221>  MISC_FEATURE
<222>  (1)..(1)
<223>  X represents (Leu)m, wherein m is 0 or 1

<220>
<221>  MISC_FEATURE
<222>  (1)..(1)
<223>  a N-terminal end corresponds to -NHR5, wherein R5 is selected
       from the group consisting of -H and (C1-C20)alkyl

<220>
<221>  MISC_FEATURE
<222>  (2)..(2)
<223>  X represents (Asp)n, wherein n is 0 or 1

<220>
<221>  MISC_FEATURE
<222>  (5)..(5)
<223>  the amino acid is of formula (II), the alpha carbon of this amino
       acid being connected to the alpha carbon of the amino acid at
       position 12 by a linker "L"

<220>
<221>  MISC_FEATURE
<222>  (12)..(12)
<223>  the amino acid is of formula (II), the alpha carbon of this amino
       acid being connected to the alpha carbon of the amino acid at
       position 5 by a linker "L"

<220>
<221>  MISC_FEATURE
<222>  (17)..(17)
<223>  X represents (Arg)p, wherein p is 0 or 1

<220>
<221>  MISC_FEATURE
<222>  (18)..(18)
<223>  X represents (Lys)q, wherein q is 0 or 1

<220>
<221>  MISC_FEATURE
<222>  (18)..(18)
<223>  C-terminal end corresponds to -C(O)R4, wherein R4 is a radical
       selected from the group consisting of -OH and -NR17R18;

<400>  2

Xaa Xaa Lys Ala Ser Val Met Arg Leu Thr Ile Ser Tyr Leu Arg Val
1               5                   10                  15


Xaa Xaa

<210>  3
<211>  18
<212>  PRT
<213>  Artificial Sequence

<220>
<223>  peptide


<220>
<221>  MISC_FEATURE
<222>  (1)..(1)
<223>  X represents (Leu)m, wherein m is 0 or 1

<220>
<221>  MISC_FEATURE
<222>  (1)..(1)
<223>  N-terminal end corresponds to -NHR5, wherein R5 is selected from
       the group consisting of -H and (C1-C20)alkyl

<220>
<221>  MISC_FEATURE
<222>  (2)..(2)
<223>  X represents (Asp)n, wherein n is 0 or 1

<220>
<221>  MISC_FEATURE
<222>  (7)..(7)
<223>  the amino acid is of formula (II), the alpha carbon of this amino
       acid being connected to the alpha carbon of the amino acid at
       position 14 by a linker "L"

<220>
<221>  MISC_FEATURE
<222>  (14)..(14)
<223>  the amino acid is of formula (II), the alpha carbon of this amino
       acid being connected to the alpha carbon of the amino acid at
       position 7 by a linker "L"

<220>
<221>  MISC_FEATURE
<222>  (17)..(17)
<223>  X represents (Arg)p, wherein p is 0 or 1

<220>
<221>  MISC_FEATURE
<222>  (18)..(18)
<223>  X represents (Lys)q, wherein q is 0 or 1

<220>
<221>  MISC_FEATURE
<222>  (18)..(18)
<223>  C-terminal end corresponds to -C(O)R4, wherein R4 is a radical
       selected from the group consisting of -OH and -NR17R18

<400>  3

Xaa Xaa Lys Ala Ser Val Met Arg Leu Thr Ile Ser Tyr Leu Arg Val
1               5                   10                  15

Xaa Xaa

<210> 4
<211> 18
<212> PRT
<213> Artificial Sequence

<220>
<223> peptide

<220>
<221> MISC_FEATURE
<222> (1)..(1)
<223> X represents (Leu)m, wherein m is 0 or 1

<220>
<221> MISC_FEATURE
<222> (1)..(1)
<223> N-terminal end corresponds to -NHR5, wherein R5 is selected from
      the group consisting of -H and (C1-C20)alkyl

<220>
<221> MISC_FEATURE
<222> (2)..(2)
<223> X represents (Asp)n, wherein n is 0 or 1

<220>
<221> MISC_FEATURE
<222> (8)..(8)
<223> the amino acid is of formula (II), the alpha carbon of this amino
      acid being connected to the alpha carbon of the amino acid at
      position 15 by a linker "L"

<220>
<221> MISC_FEATURE
<222> (15)..(15)
<223> the amino acid is of formula (II), the alpha carbon of this amino
      acid being connected to the alpha carbon of the amino acid at
      position 8 by a linker "L"

<220>
<221> MISC_FEATURE
<222> (17)..(17)
<223> X represents (Arg)p, wherein p is 0 or 1

<220>
<221> MISC_FEATURE
<222> (18)..(18)
<223> X represents (Lys)q, wherein q is 0 or 1

<220>
<221> MISC_FEATURE
<222> (18)..(18)
<223> C-terminal end corresponds to -C(O)R4, wherein R4 is a radical
      selected from the group consisting of -OH and -NR17R18

<400> 4

Xaa Xaa Lys Ala Ser Val Met Arg Leu Thr Ile Ser Tyr Leu Arg Val
1               5                   10                  15

Xaa Xaa

<210> 5
<211> 18
<212> PRT
<213> Artificial Sequence

<220>
<223> peptide

<220>
<221> MISC_FEATURE
<222> (1)..(1)
<223> X represents (Leu)m, wherein m is 0 or 1

<220>
<221> MISC_FEATURE
<222> (1)..(1)
<223> N-terminal end corresponds to -NHR5, wherein R5 is selected from
      the group consisting of -H and (C1-C20)alkyl

<220>
<221> MISC_FEATURE
<222> (2)..(2)
<223> X represents (Asp)n, wherein n is 0 or 1

<220>
<221> MISC_FEATURE
<222> (10)..(10)
<223> the amino acid is of formula (II), the alpha carbon of this amino
      acid being connected to the alpha carbon of the amino acid at
      position 17 by a linker "L"

<220>
<221> MISC_FEATURE
<222> (17)..(17)
<223> the amino acid is of formula (II), the alpha carbon of this amino
      acid being connected to the alpha carbon of the amino acid at
      position 10 by a linker "L"

<220>
<221> MISC_FEATURE
<222> (18)..(18)
<223> X represents (Lys)q, wherein q is 0 or 1

<220>
<221> MISC_FEATURE
<222> (18)..(18)
<223> C-terminal end corresponds to -C(O)R4, wherein R4 is a radical
      selected from the group consisting of -OH and -NR17R18

<400> 5

Xaa Xaa Lys Ala Ser Val Met Arg Leu Thr Ile Ser Tyr Leu Arg Val
1               5                   10                  15

Arg Xaa

<210>  6
<211>  15
<212>  PRT
<213>  Artificial Sequence

<220>
<223>  peptide

<220>
<221>  MISC_FEATURE
<222>  (1)..(1)
<223>  N-terminal end corresponds to -NHR5, wherein R5 is selected from
       the group consisting of -H and (C1-C20)alkyl

<220>
<221>  MISC_FEATURE
<222>  (3)..(3)
<223>  the amino acid is of formula (II), the alpha carbon of this amino
       acid being connected to the alpha carbon of the amino acid at
       position 10 by a linker "L"

<220>
<221>  MISC_FEATURE
<222>  (10)..(10)
<223>  the amino acid is of formula (II), the alpha carbon of this amino
       acid being connected to the alpha carbon of the amino acid at
       position 3 by a linker "L"

<220>
<221>  MISC_FEATURE
<222>  (15)..(15)
<223>  C-terminal end corresponds to -C(O)R4, wherein R4 is a radical
       selected from the group consisting of -OH and -NR17R18

<400>  6

Lys Ala Ser Val Met Arg Leu Thr Ile Ser Tyr Leu Arg Val Arg
1               5                   10                  15

<210>  7
<211>  15
<212>  PRT
<213>  Artificial Sequence

<220>
<223>  peptide

<220>
<221>  MISC_FEATURE
<222>  (1)..(1)
<223>  N-terminal end corresponds to -NHR5, wherein R5 is selected from
       the group consisting of -H and (C1-C20)alkyl

<220>
<221>  MISC_FEATURE
<222>  (5)..(5)
<223>  the amino acid is of formula (II), the alpha carbon of this amino

acid being connected to the alpha carbon of the amino acid at
      position 12 by a linker "L"

<220>
<221>  MISC_FEATURE
<222>  (12)..(12)
<223>  the amino acid is of formula (II), the alpha carbon of this amino
      acid being connected to the alpha carbon of the amino acid at
      position 5 by a linker "L"

<220>
<221>  MISC_FEATURE
<222>  (15)..(15)
<223>  C-terminal end corresponds to -C(O)R4, wherein R4 is a radical
      selected from the group consisting of -OH and -NR17R18

<400>  7

Lys Ala Ser Val Met Arg Leu Thr Ile Ser Tyr Leu Arg Val Arg
1               5                   10                  15


<210>  8
<211>  15
<212>  PRT
<213>  Artificial Sequence

<220>
<223>  peptide


<220>
<221>  MISC_FEATURE
<222>  (1)..(1)
<223>  N-terminal end corresponds to -NHR5, wherein R5 is selected from
      the group consisting of -H and (C1-C20)alkyl

<220>
<221>  MISC_FEATURE
<222>  (6)..(6)
<223>  the amino acid is of formula (II), the alpha carbon of this amino
      acid being connected to the alpha carbon of the amino acid at
      position 13 by a linker "L"

<220>
<221>  MISC_FEATURE
<222>  (13)..(13)
<223>  the amino acid is of formula (II), the alpha carbon of this amino
      acid being connected to the alpha carbon of the amino acid at
      position 6 by a linker "L"

<220>
<221>  MISC_FEATURE
<222>  (15)..(15)
<223>  C-terminal end corresponds to -C(O)R4, wherein R4 is a radical
      selected from the group consisting of -OH and -NR17R18

<400>  8

Lys Ala Ser Val Met Arg Leu Thr Ile Ser Tyr Leu Arg Val Arg
1               5                   10                  15

<210>  9
<211>  15
<212>  PRT
<213>  Artificial Sequence

<220>
<223>  peptide



<220>
<221>  MISC_FEATURE
<222>  (1)..(1)
<223>  N-terminal end corresponds to -NHR5, wherein R5 is selected from
       the group consisting of -H and (C1-C20)alkyl

<220>
<221>  MISC_FEATURE
<222>  (8)..(8)
<223>  the amino acid is of formula (II), the alpha carbon of this amino
       acid being connected to the alpha carbon of the amino acid at
       position 15 by a linker "L"

<220>
<221>  MISC_FEATURE
<222>  (15)..(15)
<223>  the amino acid is of formula (II), the alpha carbon of this amino
       acid being connected to the alpha carbon of the amino acid at
       position 8 by a linker "L"

<400>  9

Lys Ala Ser Val Met Arg Leu Thr Ile Ser Tyr Leu Arg Val Arg
1               5                   10                  15



<210>  10
<211>  18
<212>  PRT
<213>  Artificial Sequence

<220>
<223>  peptide



<220>
<221>  MISC_FEATURE
<222>  (1)..(1)
<223>  N-terminal end corresponds to -NHR5, wherein R5 is selected from
       the group consisting of -H and (C1-C20)alkyl

<220>
<221>  MISC_FEATURE
<222>  (5)..(5)
<223>  the amino acid is of formula (II), the alpha carbon of this amino
       acid being connected to the alpha carbon of the amino acid at
       position 12 by a linker "L"

<220>
<221>  MISC_FEATURE
<222>  (12)..(12)
<223>  the amino acid is of formula (II), the alpha carbon of this amino
       acid being connected to the alpha carbon of the amino acid at
       position 5 by a linker "L"

<220>
<221>  MISC_FEATURE
<222>  (18)..(18)
<223>  C-terminal end corresponds to -C(O)R4, wherein R4 is a radical
       selected from the group consisting of -OH and -NR17R18

<400>  10

Leu Asp Lys Ala Ser Val Met Arg Leu Thr Ile Ser Tyr Leu Arg Val
1               5                   10                  15


Arg Lys




<210>  11
<211>  18
<212>  PRT
<213>  Artificial Sequence

<220>
<223>  peptide



<220>
<221>  MISC_FEATURE
<222>  (1)..(1)
<223>  N-terminal end corresponds to -NHR5, wherein R5 is selected from
       the group consisting of -H and (C1-C20)alkyl

<220>
<221>  MISC_FEATURE
<222>  (7)..(7)
<223>  the amino acid is of formula (II), the alpha carbon of this amino
       acid being connected to the alpha carbon of the amino acid at
       position 12 by a linker "L"

<220>
<221>  MISC_FEATURE
<222>  (14)..(14)
<223>  the amino acid is of formula (II), the alpha carbon of this amino
       acid being connected to the alpha carbon of the amino acid at
       position 7 by a linker "L"

<220>
<221>  MISC_FEATURE
<222>  (18)..(18)
<223>  C-terminal end corresponds to -C(O)R4, wherein R4 is a radical
       selected from the group consisting of -OH and -NR17R18

<400>  11

Leu Asp Lys Ala Ser Val Met Arg Leu Thr Ile Ser Tyr Leu Arg Val
1               5                   10                  15


Arg Lys




<210>  12

<211>  18
<212>  PRT
<213>  Artificial Sequence

<220>
<223>  peptide


<220>
<221>  MISC_FEATURE
<222>  (1)..(1)
<223>  N-terminal end corresponds to -NHR5, wherein R5 is selected from
       the group consisting of -H and (C1-C20)alkyl

<220>
<221>  MISC_FEATURE
<222>  (8)..(8)
<223>  the amino acid is of formula (II), the alpha carbon of this amino
       acid being connected to the alpha carbon of the amino acid at
       position 15 by a linker "L"

<220>
<221>  MISC_FEATURE
<222>  (15)..(15)
<223>  the amino acid is of formula (II), the alpha carbon of this amino
       acid being connected to the alpha carbon of the amino acid at
       position 8 by a linker "L"

<220>
<221>  MISC_FEATURE
<222>  (18)..(18)
<223>  C-terminal end corresponds to -C(O)R4, wherein R4 is a radical
       selected from the group consisting of -OH and -NR17R18

<400>  12

Leu Asp Lys Ala Ser Val Met Arg Leu Thr Ile Ser Tyr Leu Arg Val
1               5                   10                  15


Arg Lys




<210>  13
<211>  18
<212>  PRT
<213>  Artificial Sequence

<220>
<223>  peptide

<400>  13

Leu Asp Lys Ala Ser Val Met Arg Leu Thr Ile Ser Tyr Leu Arg Val
1               5                   10                  15


Arg Lys



<210>  14

<211>  15
<212>  PRT
<213>  Artificial Sequence

<220>
<223>  peptide


<220>
<221>  MISC_FEATURE
<222>  (3)..(3)
<223>  the amino acid is of formula (II), being R19 a -CH3 radical; the
       alpha carbon of this amino acid being connected to the alpha
       carbon of amino acid at position 10 by the linker
       -(CH2)6-CH=CH-(CH2)3-

<220>
<221>  MISC_FEATURE
<222>  (10)..(10)
<223>  the amino acid is of formula (II), being R19 a -CH3 radical; the
       alpha carbon of this amino acid being connected to the alpha
       carbon of amino acid at position 3 by the linker
       -(CH2)6-CH=CH-(CH2)3-

<220>
<221>  MISC_FEATURE
<222>  (15)..(15)
<223>  C-terminal end corresponds to -C(O)NH2

<400>  14

Lys Ala Ser Val Met Arg Leu Thr Ile Ser Tyr Leu Arg Val Arg
1               5                   10                  15


<210>  15
<211>  15
<212>  PRT
<213>  Artificial Sequence

<220>
<223>  peptide


<220>
<221>  MISC_FEATURE
<222>  (5)..(5)
<223>  the amino acid is of formula (II), being R19 a -CH3 radical; the
       alpha carbon of this amino acid being connected to the alpha
       carbon of amino acid at position 12  by the linker
       -(CH2)6-CH=CH-(CH2)3-

<220>
<221>  MISC_FEATURE
<222>  (12)..(12)
<223>  the amino acid is of formula (II), being R19 a -CH3 radical; the
       alpha carbon of this amino acid being connected to the alpha
       carbon of amino acid at position 5 by the linker
       -(CH2)6-CH=CH-(CH2)3-

<220>
<221>  MISC_FEATURE
<222>  (15)..(15)

<223>  C-terminal end corresponds to -C(O)NH2

<400>  15

Lys Ala Ser Val Met Arg Leu Thr Ile Ser Tyr Leu Arg Val Arg
1               5                   10                  15


<210>  16
<211>  15
<212>  PRT
<213>  Artificial Sequence

<220>
<223>  peptide


<220>
<221>  MISC_FEATURE
<222>  (6)..(6)
<223>  the amino acid is of formula (II), being R19 a -CH3 radical; the
       alpha carbon of this amino acid being connected to the alpha
       carbon of amino acid at position 13 by the linker
       -(CH2)6-CH=CH-(CH2)3-

<220>
<221>  MISC_FEATURE
<222>  (13)..(13)
<223>  the amino acid is of formula (II), being R19 a -CH3 radical; the
       alpha carbon of this amino acid being connected to the alpha
       carbon of amino acid at position 6 by the linker
       -(CH2)6-CH=CH-(CH2)3-

<220>
<221>  MISC_FEATURE
<222>  (15)..(15)
<223>  C-terminal end corresponds to -C(O)NH2

<400>  16

Lys Ala Ser Val Met Arg Leu Thr Ile Ser Tyr Leu Arg Val Arg
1               5                   10                  15


<210>  17
<211>  15
<212>  PRT
<213>  Artificial Sequence

<220>
<223>  peptide


<220>
<221>  MISC_FEATURE
<222>  (6)..(6)
<223>  the amino acid is of formula (II), being R19 a -CH3 radical; the
       alpha carbon of this amino acid being connected to the alpha
       carbon of amino acid at position 13 by the linker
       -(CH2)6-CH=CH-(CH2)3-

<220>
<221>  MISC_FEATURE

<222>   (13)..(13)
<223>   the amino acid is of formula (II), being R19 a -CH3 radical; the
        alpha carbon of this amino acid being connected to the alpha
        carbon of amino acid at position 6 by the linker
        -(CH2)6-CH=CH-(CH2)3-

<400>   17

Lys Ala Ser Val Met Arg Leu Thr Ile Ser Tyr Leu Arg Val Arg
1               5                   10                  15


<210>   18
<211>   15
<212>   PRT
<213>   Artificial Sequence

<220>
<223>   peptide


<220>
<221>   MISC_FEATURE
<222>   (8)..(8)
<223>   the amino acid is of formula (II), being R19 a -CH3 radical; the
        alpha carbon of this amino acid being connected to the alpha
        carbon of amino acid at position 15 by the linker
        -(CH2)6-CH=CH-(CH2)3-

<220>
<221>   MISC_FEATURE
<222>   (15)..(15)
<223>   the amino acid is of formula (II), being R19 a -CH3 radical; the
        alpha carbon of this amino acid being connected to the alpha
        carbon of amino acid at position 8 by the linker
        -(CH2)6-CH=CH-(CH2)3-

<220>
<221>   MISC_FEATURE
<222>   (15)..(15)
<223>   C-terminal end corresponds to -C(O)NH2

<400>   18

Lys Ala Ser Val Met Arg Leu Thr Ile Ser Tyr Leu Arg Val Arg
1               5                   10                  15


<210>   19
<211>   18
<212>   PRT
<213>   Artificial Sequence

<220>
<223>   peptide


<220>
<221>   MISC_FEATURE
<222>   (5)..(5)
<223>   the amino acid is of formula (II), being R19 a -CH3 radical; the
        alpha carbon of this amino acid being connected to the alpha
        carbon of amino acid at position 12 by the linker

-(CH2)6-CH=CH-(CH2)3-

<220>
<221> MISC_FEATURE
<222> (12)..(12)
<223> the amino acid is of formula (II), being R19 a -CH3 radical; the
      alpha carbon of this amino acid being connected to the alpha
      carbon of amino acid at position 5 by the linker
      -(CH2)6-CH=CH-(CH2)3-

<220>
<221> MISC_FEATURE
<222> (18)..(18)
<223> C-terminal end corresponds to -C(O)NH2

<400> 19

Leu Asp Lys Ala Ser Val Met Arg Leu Thr Ile Ser Tyr Leu Arg Val
1               5                   10                  15

Arg Lys

<210> 20
<211> 18
<212> PRT
<213> Artificial Sequence

<220>
<223> peptide

<220>
<221> MISC_FEATURE
<222> (7)..(7)
<223> the amino acid is of formula (II), being R19 a -CH3 radical; the
      alpha carbon of this amino acid being connected to the alpha
      carbon of amino acid at position 14 by the linker
      -(CH2)6-CH=CH-(CH2)3-

<220>
<221> MISC_FEATURE
<222> (14)..(14)
<223> the amino acid is of formula (II), being R19 a -CH3 radical; the
      alpha carbon of this amino acid being connected to the alpha
      carbon of amino acid at position 7 by the linker
      -(CH2)6-CH=CH-(CH2)3-

<220>
<221> MISC_FEATURE
<222> (18)..(18)
<223> C-terminal end corresponds to -C(O)NH2

<400> 20

Leu Asp Lys Ala Ser Val Met Arg Leu Thr Ile Ser Tyr Leu Arg Val
1               5                   10                  15

Arg Lys

<210>  21
<211>  18
<212>  PRT
<213>  Artificial Sequence

<220>
<223>  peptide


<220>
<221>  MISC_FEATURE
<222>  (8)..(8)
<223>  the amino acid is of formula (II), being R19 a -CH3 radical; the
       alpha carbon of this amino acid being connected to the alpha
       carbon of amino acid at position 15 by the linker
       -(CH2)6-CH=CH-(CH2)3-

<220>
<221>  MISC_FEATURE
<222>  (15)..(15)
<223>  the amino acid is of formula (II), being R19 a -CH3 radical; the
       alpha carbon of this amino acid being connected to the alpha
       carbon of amino acid at position 8 by the linker
       -(CH2)6-CH=CH-(CH2)3-

<220>
<221>  MISC_FEATURE
<222>  (18)..(18)
<223>  C-terminal end corresponds to -C(O)NH2

<400>  21

Leu Asp Lys Ala Ser Val Met Arg Leu Thr Ile Ser Tyr Leu Arg Val
1               5                   10                  15


Arg Lys




<210>  22
<211>  19
<212>  PRT
<213>  Artificial Sequence

<220>
<223>  peptide


<220>
<221>  MISC_FEATURE
<222>  (1)..(1)
<223>  X represents (Arg)r, wherein r is 0 or 1

<220>
<221>  MISC_FEATURE
<222>  (1)..(1)
<223>  N-terminal end corresponds to -NHR5, wherein R5 is selected from
       the group consisting of -H and (C1-C20)alkyl

<220>

<221>  MISC_FEATURE
<222>  (2)..(2)
<223>  X represents (Ser)s, wherein s is 0 or 1

<220>
<221>  MISC_FEATURE
<222>  (18)..(18)
<223>  X represents (Leu)t, wherein t is 0 or 1

<220>
<221>  MISC_FEATURE
<222>  (19)..(19)
<223>  X represents (Pro)v, wherein v is 0 or 1

<220>
<221>  MISC_FEATURE
<222>  (19)..(19)
<223>  C-terminal end corresponds to -C(O)R4, wherein R4 is a radical
       selected from the group consisting of -OH and -NR17R18

<400>  22

Xaa Xaa Arg Arg Ser Lys Glu Ser Glu Val Phe Tyr Glu Leu Ala His
1               5                   10                  15


Gln Xaa Xaa




<210>  23
<211>  19
<212>  PRT
<213>  Artificial Sequence

<220>
<223>  peptide



<220>
<221>  MISC_FEATURE
<222>  (1)..(1)
<223>  X represents (Arg)r, wherein r is 0 or 1

<220>
<221>  MISC_FEATURE
<222>  (1)..(1)
<223>  N-terminal end corresponds to -NHR5, wherein R5 is selected from
       the group consisting of -H and (C1-C20)alkyl

<220>
<221>  MISC_FEATURE
<222>  (2)..(2)
<223>  X represents (Ser)s, wherein s is 0 or 1

<220>
<221>  MISC_FEATURE
<222>  (8)..(8)
<223>  the amino acid is of formula (II), the alpha carbon of this amino
       acid being connected to the alpha carbon of amino acid at
       position 15 by the linker "L"

<220>

<221>  MISC_FEATURE
<222>  (15)..(15)
<223>  the amino acid is of formula (II), the alpha carbon of this amino
       acid being connected to the alpha carbon of amino acid at
       position 8 by the linker "L"

<220>
<221>  MISC_FEATURE
<222>  (18)..(18)
<223>  X represents (Leu)t, wherein t is 0 or 1

<220>
<221>  MISC_FEATURE
<222>  (19)..(19)
<223>  X represents (Pro)v, wherein v is 0 or 1

<220>
<221>  MISC_FEATURE
<222>  (19)..(19)
<223>  C-terminal end corresponds to -C(O)R4, wherein R4 is a radical
       selected from the group consisting of -OH and -NR17R18

<400>  23

Xaa Xaa Arg Arg Ser Lys Glu Ser Glu Val Phe Tyr Glu Leu Ala His
1               5                   10                  15

Gln Xaa Xaa

<210>  24
<211>  19
<212>  PRT
<213>  Artificial Sequence

<220>
<223>  peptide

<220>
<221>  MISC_FEATURE
<222>  (1)..(1)
<223>  X represents (Arg)r, wherein r is 0 or 1

<220>
<221>  MISC_FEATURE
<222>  (1)..(1)
<223>  N-terminal end corresponds to -NHR5, wherein R5 is selected from
       the group consisting of -H and (C1-C20)alkyl

<220>
<221>  MISC_FEATURE
<222>  (2)..(2)
<223>  X represents (Ser)s, wherein s is 0 or 1

<220>
<221>  MISC_FEATURE
<222>  (10)..(10)
<223>  the amino acid is of formula (II), the alpha carbon of this amino
       acid being connected to the alpha carbon of amino acid at
       position 17  by the linker "L"

<220>
<221>  MISC_FEATURE
<222>  (17)..(17)
<223>  the amino acid is of formula (II), the alpha carbon of this amino
       acid being connected to the alpha carbon of amino acid at
       position 10  by the linker "L"

<220>
<221>  MISC_FEATURE
<222>  (18)..(18)
<223>  X represents (Leu)t, wherein t is 0 or 1

<220>
<221>  MISC_FEATURE
<222>  (19)..(19)
<223>  X represents (Pro)v, wherein v is 0 or 1

<220>
<221>  MISC_FEATURE
<222>  (19)..(19)
<223>  C-terminal end corresponds to -C(O)R4, wherein R4 is a radical
       selected from the group consisting of -OH and -NR17R18

<400>  24

Xaa Xaa Arg Arg Ser Lys Glu Ser Glu Val Phe Tyr Glu Leu Ala His
1               5                   10                  15


Gln Xaa Xaa




<210>  25
<211>  19
<212>  PRT
<213>  Artificial Sequence

<220>
<223>  peptide


<220>
<221>  MISC_FEATURE
<222>  (1)..(1)
<223>  X represents (Arg)r, wherein r is 0 or 1

<220>
<221>  MISC_FEATURE
<222>  (1)..(1)
<223>  N-terminal end corresponds to -NHR5, wherein R5 is selected from
       the group consisting of -H and (C1-C20)alkyl

<220>
<221>  MISC_FEATURE
<222>  (2)..(2)
<223>  X represents (Ser)s, wherein s is 0 or 1

<220>
<221>  MISC_FEATURE
<222>  (5)..(5)
<223>  the amino acid is of formula (II), the alpha carbon of this amino

acid being connected to the alpha carbon of amino acid at
position 12 by the linker "L"

<220>
<221> MISC_FEATURE
<222> (12)..(12)
<223> the amino acid is of formula (II), the alpha carbon of this amino
acid being connected to the alpha carbon of amino acid at
position 5 by the linker "L"

<220>
<221> MISC_FEATURE
<222> (18)..(18)
<223> X represents (Leu)t, wherein t is 0 or 1

<220>
<221> MISC_FEATURE
<222> (19)..(19)
<223> X represents (Pro)v, wherein v is 0 or 1

<220>
<221> MISC_FEATURE
<222> (19)..(19)
<223> C-terminal end corresponds to -C(O)R4, wherein R4 is a radical
selected from the group consisting of -OH and -NR17R18

<400> 25

Xaa Xaa Arg Arg Ser Lys Glu Ser Glu Val Phe Tyr Glu Leu Ala His
1               5                   10                  15

Gln Xaa Xaa

<210> 26
<211> 17
<212> PRT
<213> Artificial Sequence

<220>
<223> peptide

<220>
<221> MISC_FEATURE
<222> (1)..(1)
<223> N-terminal end corresponds to -NHR5, wherein R5 is selected from
the group consisting of -H and (C1-C20)alkyl

<220>
<221> MISC_FEATURE
<222> (8)..(8)
<223> the amino acid is of formula (II), the alpha carbon of this amino
acid being connected to the alpha carbon of amino acid at
position 15 by the linker "L"

<220>
<221> MISC_FEATURE
<222> (15)..(15)
<223> the amino acid is of formula (II), the alpha carbon of this amino
acid being connected to the alpha carbon of amino acid at

position 8 by the linker "L"

<220>
<221>  MISC_FEATURE
<222>  (17)..(17)
<223>  C-terminal end corresponds to -C(O)R4, wherein R4 is a radical
       selected from the group consisting of -OH and -NR17R18

<400>  26

Arg Ser Arg Arg Ser Lys Glu Ser Glu Val Phe Tyr Glu Leu Ala His
1               5                   10                  15

Gln

<210>  27
<211>  17
<212>  PRT
<213>  Artificial Sequence

<220>
<223>  peptide

<220>
<221>  MISC_FEATURE
<222>  (1)..(1)
<223>  N-terminal end corresponds to -NHR5, wherein R5 is selected from
       the group consisting of -H and (C1-C20)alkyl

<220>
<221>  MISC_FEATURE
<222>  (10)..(10)
<223>  the amino acid is of formula (II), the alpha carbon of this amino
       acid being connected to the alpha carbon of amino acid at
       position 17 by the linker "L"

<220>
<221>  MISC_FEATURE
<222>  (17)..(17)
<223>  the amino acid is of formula (II), the alpha carbon of this amino
       acid being connected to the alpha carbon of amino acid at
       position 10 by the linker "L"

<220>
<221>  MISC_FEATURE
<222>  (17)..(17)
<223>  C-terminal end corresponds to -C(O)R4, wherein R4 is a radical
       selected from the group consisting of -OH and -NR17R18

<400>  27

Arg Ser Arg Arg Ser Lys Glu Ser Glu Val Phe Tyr Glu Leu Ala His
1               5                   10                  15

Gln

<210>  28
<211>  19
<212>  PRT
<213>  Artificial Sequence

<220>
<223>  peptide



<220>
<221>  MISC_FEATURE
<222>  (1)..(1)
<223>  N-terminal end corresponds to -NHR5, wherein R5 is selected from
       the group consisting of -H and (C1-C20)alkyl

<220>
<221>  MISC_FEATURE
<222>  (3)..(3)
<223>  the amino acid is of formula (II), the alpha carbon of this amino
       acid being connected to the alpha carbon of amino acid at
       position 10 by the linker "L"

<220>
<221>  MISC_FEATURE
<222>  (10)..(10)
<223>  the amino acid is of formula (II), the alpha carbon of this amino
       acid being connected to the alpha carbon of amino acid at
       position 3 by the linker "L"

<220>
<221>  MISC_FEATURE
<222>  (19)..(19)
<223>  C-terminal end corresponds to -C(O)R4, wherein R4 is a radical
       selected from the group consisting of -OH and -NR17R18

<400>  28

Arg Ser Arg Arg Ser Lys Glu Ser Glu Val Phe Tyr Glu Leu Ala His
1               5                   10                  15


Gln Leu Pro




<210>  29
<211>  17
<212>  PRT
<213>  Artificial Sequence

<220>
<223>  peptide



<220>
<221>  MISC_FEATURE
<222>  (8)..(8)
<223>  the amino acid is of formula (II), being R19 a -CH3 radical; the
       alpha carbon of this amino acid being connected to the alpha
       carbon of amino acid at position 15 by the linker
       -(CH2)6-CH=CH-(CH2)3-

<220>

<221>  MISC_FEATURE
<222>  (15)..(15)
<223>  the amino acid is of formula (II), being R19 a -CH3 radical; the
       alpha carbon of this amino acid being connected to the alpha
       carbon of amino acid at position 8 by the linker
       -(CH2)6-CH=CH-(CH2)3-

<220>
<221>  MISC_FEATURE
<222>  (17)..(17)
<223>  C-terminal end corresponds to -C(O)NH2

<400>  29

Arg Ser Arg Arg Ser Lys Glu Ser Glu Val Phe Tyr Glu Leu Ala His
1               5                   10                  15

Gln

<210>  30
<211>  17
<212>  PRT
<213>  Artificial Sequence

<220>
<223>  peptide

<220>
<221>  MISC_FEATURE
<222>  (10)..(10)
<223>  the amino acid is of formula (II), being R19 a -CH3 radical; the
       alpha carbon of this amino acid being connected to the alpha
       carbon of amino acid at position 17 by the linker
       -(CH2)6-CH=CH-(CH2)3-

<220>
<221>  MISC_FEATURE
<222>  (17)..(17)
<223>  the amino acid is of formula (II), being R19 a -CH3 radical; the
       alpha carbon of this amino acid being connected to the alpha
       carbon of amino acid at position 10 by the linker
       -(CH2)6-CH=CH-(CH2)3-

<220>
<221>  MISC_FEATURE
<222>  (17)..(17)
<223>  C-terminal end corresponds to -C(O)NH2

<400>  30

Arg Ser Arg Arg Ser Lys Glu Ser Glu Val Phe Tyr Glu Leu Ala His
1               5                   10                  15

Gln

<210>  31

<211>  19
<212>  PRT
<213>  Artificial Sequence

<220>
<223>  peptide

<220>
<221>  MISC_FEATURE
<222>  (3)..(3)
<223>  the amino acid is of formula (II), being R19 a -CH3 radical; the
       alpha carbon of this amino acid being connected to the alpha
       carbon of amino acid at position 10 by the linker
       -(CH2)6-CH=CH-(CH2)3-

<220>
<221>  MISC_FEATURE
<222>  (10)..(10)
<223>  the amino acid is of formula (II), being R19 a -CH3 radical; the
       alpha carbon of this amino acid being connected to the alpha
       carbon of amino acid at position 3 by the linker
       -(CH2)6-CH=CH-(CH2)3-

<220>
<221>  MISC_FEATURE
<222>  (19)..(19)
<223>  C-terminal end corresponds to -C(O)NH2

<400>  31

Arg Ser Arg Arg Ser Lys Glu Ser Glu Val Phe Tyr Glu Leu Ala His
1               5                   10                  15

Gln Leu Pro

<210>  32
<211>  20
<212>  DNA
<213>  Artificial Sequence

<220>
<223>  primer

<400>  32
agaaaatctg gcaccacacc                                                20

<210>  33
<211>  20
<212>  DNA
<213>  Artificial Sequence

<220>
<223>  primer

<400>  33
ggggtgttga aggtctcaaa                                                20

<210>  34
<211>  20
<212>  DNA
<213>  Artificial Sequence

<220>
<223>  primer

<400>  34
tactgccatc caatcgagac                                                    20


<210>  35
<211>  18
<212>  DNA
<213>  Artificial Sequence

<220>
<223>  primer

<400>  35
gcatggtgat gttggact                                                      18

## Description

### Technical Field

The present invention refers to the fields of the peptides and therapy. In particular, the present invention provides peptides with the ability of regulating VEGF protein. This makes them useful in the regulation of processes wherein VEGF plays an essential role, such as the angiogenesis. The invention also provides fusion proteins and pharmaceutical compositions comprising the peptides of the invention.

### Background Art

Angiogenesis plays a critical role in a wide variety of fundamental physiological processes in the normal individual including embryogenesis, somatic growth, and differentiation of the nervous system. In the female reproductive system, angiogenesis occurs in the follicle during its development, in the corpus luteum following ovulation and in the placenta to establish and maintain pregnancy. Angiogenesis additionally occurs as part of the body's repair processes, such as in the healing of wounds and fractures. Thus, promotion of angiogenesis can be useful in situations in which establishment or extension of vascularization is desirable. Angiogenesis, however, is also a critical factor in a number of pathological processes, perhaps most notably tumor growth and metastasis, as tumors require continuous stimulation of new capillary blood vessels in order to grow. Other pathological processes affected by angiogenesis include conditions associated with blood vessel proliferation, especially in the capillaries, such as diabetic retinopathy, macular degeneration, arthropathies, psoriasis, rheumatoid arthritis, pulmonary hypertension, chronic obstructive pulmonary disease, liver disease, renal disease etc (Carmeliet P., 2003).

Current research indicates that a family of endothelial cell-specific growth factors, the vascular endothelial growth factors (VEGFs), together with their cognate receptors, are primarily responsible for stimulation of endothelial cell growth and differentiation. These factors are members of the PDGF family and appear to act primarily via endothelial receptor tyrosine kinases (RTKs).

A crucial mode of vessel formation during both embryonic development and adult life, sprouting angiogenesis forms new vessels by the outgrowth of endothelial cells from existing vessels. As endothelial cells are normally quiescent, a stimulating cytokine, such as VEGF, is required to commence angiogenesis. VEGF, considered a master regulator of angiogenesis in its own right, causes endothelial cells to detach from the parent vessel and migrate into the neighbouring stroma. Hypoxia is the principal regulator of VEGF expression, as it is a direct transcriptional target of both HIF-1α and HIF-2α. Arnt mutant vascular defects are accompanied by decreased VEGF expression, which is responsible for many of the vascular anomalies observed, as exogenous VEGF rescues these defects.

Occlusive vascular diseases remain the most important causes of death and morbidity in industrialized societies. Treatment of end stages such as myocardial infarction, peripheral artery disease (PAD), and stroke is usually limited only to palliative interventions, such as angioplasty and, in severe PAD cases, limb amputation. The ability to induce and regulate angiogenesis and vascular remodelling in a directed manner would represent a major advance in the treatment of ischemic vascular diseases On the other hand, a further example of uncontrolled angiogenesis with dramatical effects on health, is the uncontrolled blood vessel growth, which has become a central pathological component of many human blindness disorders, including diabetic retinopathy, age-related macular degeneration (AMD), glaucoma, and retinopathy of prematurity (ROP). Neuronal cell death and vision loss observed in these diseases are caused by aberrant, leaky vessels, which are often associated with pathological neovascularization. Collectively, these diseases are the most common cause of vision loss, suggesting that highly effective anti-angiogenic therapy would have a tremendous effect on the prevalence of blindness in the industrialized world. In fact, neovascular eye diseases are the clearest example of the therapeutic utility of blocking angiogenesis, as VEGF inhibitors have been successfully employed in the clinic and shown efficacy in treatment of the wet (neovascular) form of AMD.

In view of the above, many efforts have been focused in finding molecules with the ability of modulating the angiogenesis, either in a positive way (i.e., stimulating VEGF) or in a negative way (i.e., inhibiting VEGF).

Unfortunately, VEGF-based therapies are not effective enough in appropriately regulating angiogenesis. Therefore, there remains the need of anti-VEGF therapies efficient in the regulation of angiogenesis.

### Summary of Invention

The present inventors have found peptides which efficiently regulates the expression of VEGF under hypoxia conditions.

Thus, in a first aspect the present invention provides a peptide or a pharmaceutical salt thereof having a length from 14 to 50 amino acids and comprising a sequence having at least a 85% of sequence identity with respect to SEQ ID NO: 1:
(Leu)m-(Asp)n-Lys-Ala-Ser-Val-Met-Arg-Leu-Thr-Ile-Ser-Tyr-Leu-Arg-Val-(Arg)p-(Lys)q
wherein
- "m", "n", "p", and "q" represent integers and are selected from 0 and 1;
- C-terminal end corresponds to -C(O)R₄; and
- N-terminal end corresponds to -NHR₅;
the peptide optionally comprising a linker biradical "L" of formula (I)

-[(R₁)ₐ-(R₂)-(R₃)_{b}]_{c}- (I)

which connects an alpha carbon atom of an amino acid located at position "i" with an alpha carbon atom of an amino acid located at position "i+4" or "i+7" in the peptide sequence SEQ ID NO: 1,
wherein
"a" and "b" are the same or different and are 0 or 1;
"c" is comprised from 1 to 10;
R₁ and R₃ are biradicals independently selected from the group consisting of: (C₁-C₁₀)alkyl; (C₁-C₁₀)alkyl substituted by one or more radicals selected from the group consisting of: halogen, (C₁-C₁₀)alkyl, -OR₆, -NR₇R₈, -SR₉, -SOR₁₀, -SO₂R₁₁, and -CO₂R₁₂; (C₂-C₁₀)alkenyl; (C₂-C₁₀)alkenyl substituted by one or more radicals selected from the group consisting of: halogen, (C₁-C₁₀)alkyl, - OR₆, -NR₇R₈, -SR₉, -SOR₁₀, -SO₂R₁₁, and -CO₂R₁₂; (C₂-C₁₀)alkynyl; and (C₂-C₁₀)alkynyl substituted by one or more radicals selected from the group consisting of: halogen, (C₁-C₁₀)alkyl, -OR₆, - NR₇R₈, -SR₉, -SOR₁₀, -SO₂R₁₁, and -CO₂R₁₂;
R₂ is a biradical selected from the group consisting of: -O-, C(=O), C(=O)NR₁₃, C(=O)O, S(=O), S(=O)₂, NR₁₄, (C₁-C₁₀)alkyl, (C₂-C₁₀)alkenyl, (C₂-C₁₀)alkynyl, -NR₁₅-NR₁₆-, -N=N-, -S-S-, and a known ring system comprising from 3 to 14 members, the system comprising from 1 to 3 rings, where:
   each one of the rings is saturated, partially unsaturated, or aromatic;
   the rings are isolated, partially or totally fused,
   each one of the members forming the known ring system is selected from the group consisting of: -CH-, -CH₂-, -NH-, -N-, -SH-, -S-, and -O-; and
   the ring system is optionally substituted by one or more radicals independently selected from the group consisting of halogen, -OH, -NO₂, (C₁-C₁₀)alkyl, (C₁-C₁₀)haloalkyl, and (C₁-C₁₀)alkyl-O-; and
R₄ is a radical selected from the group consisting of -OH and -NR₁₇R₁₈;
R₅ is a radical selected from the group consisting of -H and (C₁-C₂₀)alkyl;
R₆, R₇, R₈, R₉, R₁₀, R₁₁, R₁₂, R₁₃, R₁₄, R₁₅, R₁₆, R₁₇ and R₁₈ are radicals independently selected from the group consisting of: -H and (C₁-C₁₀)alkyl; and
the amino acids which are connected by the linker being of formula (II) 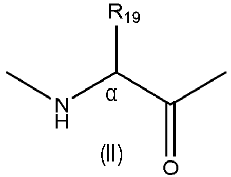
wherein
R₁₉ is a monoradical selected from the group consisting of: (C₁-C₁₀)alkyl, (C₂-C₁₀)alkenyl, (C₂-C₁₀)alkynyl, and a known ring system comprising from 3 to 14 members, the system comprising from 1 to 3 rings, where:
each one of the rings is saturated, partially unsaturated, or aromatic;
the rings are isolated, partially or totally fused,
each one of the members forming the known ring system is selected from the group consisting of: - CH-, -CH₂-, -NH-, -N-, -SH-, -S-, and -O-.

As it is shown below, the peptides of the first aspect of the invention efficiently regulates the expression of VEGF under hypoxia conditions when compared with normoxia conditions. In particular, it is shown that when the peptide of the first aspect of the invention is lineal (i.e., does not include a linker biradical "L"), an increase in the VEGF value is obtained. Surprisingly, the present inventors found that the behaviour of the peptide of the first aspect of the invention remarkably changed when the linker biradical was included, giving rise to a dramatically decrease in VEGF expression, about 50%. Thus, the inventors have found that the peptide comprising the sequence SEQ ID NO: 1 can modulate VEGF expression and, consequently, the angiogenesis, by including or not the linker biradical "L" in the sequence SEQ ID NO: 1: the absence of the linker provides a peptide that enhances the VEGF expression, and consequently "enhances" angiogenesis, whereas the presence of the linker provides a peptide that reduces VEGF expression, and consequently "reduces" angiogenesis.

It is the first time that it is reported the possibility of tuning the regulatory effect of a peptide on VEGF expression by the presence of linkers biradicals in said peptide.

In a second aspect, the present invention provides a peptide or a pharmaceutical salt thereof having a length from 15 to 50 amino acids and comprising a sequence having at least a 85% of sequence identity with respect to SEQ ID NO: 22
(Arg)r-(Ser)s-Arg-Arg-Ser-Lys-Glu-Ser-Glu-Val-Phe-Tyr-Glu-Leu-Ala-His-Gln-(Leu)t-(Pro)v
wherein
- "r", "s", "t", and "v" represent integers and are selected from 0 and 1;
- C-terminal end corresponds to -C(O)R₄; and
- N-terminal end corresponds to -NHR₅;
the peptide comprising a linker biradical "L" of formula (I)

-[(R₁)ₐ-(R₂)-(R₃)_{b}]_{c}- (I)

which is connecting an alpha carbon atom of an amino acid located at position "i" in the peptide sequence of formula (I) with an alpha carbon atom of an amino acid located at position "i+4" or "i+7" in the peptide sequence SEQ ID NO: 22,
wherein
"a" and "b" are the same or different and are 0 or 1;
"c" is comprised from 1 to 10;
R₁ and R₃ are biradicals independently selected from the group consisting of: (C₁-C₁₀)alkyl; (C₁-C₁₀)alkyl substituted by one or more radicals selected from the group consisting of: halogen, (C₁-C₁₀)alkyl, -OR₆, -NR₇R₈, -SR₉, -SOR₁₀, -SO₂R₁₁, and -CO₂R₁₂; (C₂-C₁₀)alkenyl; (C₂-C₁₀)alkenyl substituted by one or more radicals selected from the group consisting of: halogen, (C₁-C₁₀)alkyl, - OR₆, -NR₇R₈, -SR₉, -SOR₁₀, -SO₂R₁₁, and -CO₂R₁₂; (C₂-C₁₀)alkynyl; and (C₂-C₁₀)alkynyl substituted by one or more radicals selected from the group consisting of: halogen, (C₁-C₁₀)alkyl, -OR₆, - NR₇R₈, -SR₉, -SOR₁₀, -SO₂R₁₁, and -CO₂R₁₂;
R₂ is a biradical selected from the group consisting of: -O-, C(=O), C(=O)NR₁₃, C(=O)O, S(=O), S(=O)₂, NR₁₄, (C₁-C₁₀)alkyl, (C₂-C₁₀)alkenyl, (C₂-C₁₀)alkynyl, -NR₁₅-NR₁₆-, -N=N-, -S-S-, and a known ring system comprising from 3 to 14 members, the system comprising from 1 to 3 rings, where:
   each one of the rings is saturated, partially unsaturated, or aromatic;
   the rings are isolated, partially or totally fused,
   each one of the members forming the known ring system is selected from the group consisting of: -CH-, -CH₂-, -NH-, -N-, -SH-, -S-, and -O-; and
   the ring system is optionally substituted by one or more radicals independently selected from the group consisting of halogen, -OH, -NO₂, (C₁-C₁₀)alkyl, (C₁-C₁₀)haloalkyl, and (C₁-C₁₀)alkyl-O-; and
R₄ is a radical selected from the group consisting of -OH and -NR₁₇R₁₈;
R₅ is a radical selected from the group consisting of -H and (C₁-C₂₀)alkyl;
R₆, R₇, R₈, R₉, R₁₀, R₁₁, R₁₂, R₁₃, R₁₄, R₁₅, R₁₆, R₁₇ and R₁₈ are radicals independently selected from the group consisting of: -H and (C₁-C₁₀)alkyl; and
the amino acids which are connected by the linker being of formula (II) 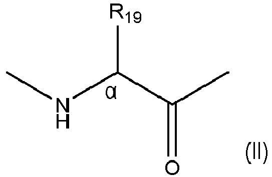 wherein
   R₁₉ is a monoradical selected from the group consisting of: (C₁-C₁₀)alkyl, (C₂-C₁₀)alkenyl, (C₂-C₁₀)alkynyl, and a known ring system comprising from 3 to 14 members, the system comprising from 1 to 3 rings, where:
   each one of the rings is saturated, partially unsaturated, or aromatic;
   the rings are isolated, partially or totally fused,
   each one of the members forming the known ring system is selected from the group consisting of: - CH-, -CH₂-, -NH-, -N-, -SH-, -S-, and -O-.

As it is shown below, the peptides of the second aspect of the invention, incorporating a linker biradical, are also capable of regulating angiogenesis. In particular, it has been found that the peptides of the second aspect of the invention are capable of increasing the expression of VEGF under hypoxia conditions with respect to the levels obtained under normoxia conditions.

Thus, the peptides of the second aspect of the invention can be useful to recover the levels of VEGF in those situations wherein a reduction in the growth factor has occurred. And, consequently, these peptides of the second aspect of the invention can find application in the prevention or treatment of diseases caused by a dysregulation of angiogenesis due to a reduction in VEGF levels.

In a third aspect, the present invention provides a fusion protein comprising the peptide as defined in the first or second aspect of the invention and, optionally, a cell penetrating peptide.

In a fourth aspect, the present invention provides a pharmaceutical composition comprising a therapeutically effective amount of the peptide or a pharmaceutical salt thereof as defined in the first or second aspect of the invention, or the fusion protein as defined in the third aspect of the invention, together with acceptable pharmaceutical excipients and/or carriers.

As it has been explained above, the peptides of the invention are able of regulating the expression of VEGF. This is indicative of the usability of the peptides of the invention in the treatment of diseases related to a dysregulation of VEGF. VEGF has been widely reported as playing a central role in the angiogenesis process. Thus, the peptides of the invention, as regulators of VEGF expression, are also capable of modulating the angiogenesis process: increasing the expression of VEGF, it is stimulated the angiogenesis, whereas if VEGF expression is reduced, then angiogenesis process is downregulated.

Thus, in a fifth aspect, the present invention provides a peptide or a pharmaceutical salt thereof as defined in the first or second aspect of the invention, or a fusion protein as defined in the third aspect of the invention or the pharmaceutical composition as defined in the fourth aspect of the invention for use as in therapy.

In a sixth aspect, the present invention provides a peptide or a pharmaceutical salt thereof as defined in the first or second aspect of the invention, or a fusion protein as defined in the third aspect of the invention, or the pharmaceutical composition as defined in the fourth aspect of the invention, for use in the treatment or prevention of a disease caused by a dysregulation in the VEGF expression level. This aspect can be alternatively be formulated as a method for the prevention or treatment of a disease caused by a dysregulation in the VEGF expression level, the method comprising the step of administering a therapeutically effective amount of a peptide or a pharmaceutical salt thereof as defined in the first or second aspect of the invention, or a fusion protein as defined in the third aspect of the invention or the pharmaceutical composition as defined in the fourth aspect of the invention, to a subject in need thereof. This aspect can be alternatively be formulated as the use of a peptide or a pharmaceutical salt thereof as defined in the first or second aspect of the invention, or a fusion protein as defined in the third aspect of the invention or the pharmaceutical composition as defined in the fourth aspect of the invention in the manufacture of a medicament for the treatment or prevention of a disease caused by a dysregulation in the VEGF expression level.

In a seventh aspect the present invention provides a peptide or a pharmaceutical salt thereof as defined in the first or second aspect of the invention, or a fusion protein as defined in the third aspect of the invention or the pharmaceutical composition as defined in the fourth aspect of the invention for use in in the treatment or prevention of a disease caused by a dysregulation of the angiogenesis, particularly by regulating the VEGF expression level. This aspect can be alternatively be reworded as a method for the treatment or prevention of a disease caused by a dysregulation of the angiogenesis, particularly by regulating the VEGF expression level, the method comprising the step of administering a therapeutically effective amount of a peptide or a pharmaceutical salt thereof as defined in the first or second aspect of the invention, or a fusion protein as defined in the third aspect of the invention or the pharmaceutical composition as defined in the fourth aspect of the invention, to a subject in need thereof. This aspect can be alternatively be formulated as the use of a peptide or a pharmaceutical salt thereof as defined in the first or second aspect of the invention, or a fusion protein as defined in the third aspect of the invention or the pharmaceutical composition as defined in the fourth aspect of the invention in the manufacture of a medicament for the treatment or prevention of a disease caused by a dysregulation of the angiogenesis, by regulating the VEGF expression level.

### Detailed description of the invention

All terms as used herein in this application, unless otherwise stated, shall be understood in their ordinary meaning as known in the art. Other more specific definitions for certain terms as used in the present application are as set forth below and are intended to apply uniformly through-out the specification and claims unless an otherwise expressly set out definition provides a broader definition.

For the purposes of the present invention, any ranges given include both the lower and the upper end-points of the range.

The present invention provides peptides comprising a sequence with at least 85% degree identity with SEQ ID NO: 1 or with SEQ ID NO: 22, as it has been stated above.

In the present invention the term "identity" refers to the percentage of residues that are identical in the two sequences when the sequences are optimally aligned. If, in the optimal alignment, a position in a first sequence is occupied by the same amino acid residue as the corresponding position in the second sequence, the sequences exhibit identity with respect to that position. The level of identity between two sequences (or "percent sequence identity") is measured as a ratio of the number of identical positions shared by the sequences with respect to the size of the sequences (i.e., percent sequence identity = (number of identical positions/total number of positions) x 100).

A number of mathematical algorithms for rapidly obtaining the optimal alignment and calculating identity between two or more sequences are known and incorporated into a number of available software programs. Examples of such programs include the MATCH-BOX, MULTAIN, GCG, FASTA, and ROBUST programs for amino acid sequence analysis, among others. Preferred software analysis programs include the ALIGN, CLUSTAL W, and BLAST programs (e.g., BLAST 2.1, BL2SEQ, and later versions thereof).

For amino acid sequence analysis, a weight matrix, such as the BLOSUM matrixes (e.g., the BLOSUM45, BLOSUM50, BLOSUM62, and BLOSUM80 matrixes), Gonnet matrixes, or PAM matrixes (e.g., the PAM30, PAM70, PAM120, PAM160, PAM250, and PAM350 matrixes), are used in determining identity.

The BLAST programs provide analysis of at least two amino acid sequences, either by aligning a selected sequence against multiple sequences in a database (e.g., GenSeq), or, with BL2SEQ, between two selected sequences. BLAST programs are preferably modified by low complexity filtering programs such as the DUST or SEG programs, which are preferably integrated into the BLAST program operations. If gap existence costs (or gap scores) are used, the gap existence cost preferably is set between about -5 and -15. Similar gap parameters can be used with other programs as appropriate. The BLAST programs and principles underlying them are further described in, e.g., Altschul et al., "Basic local alignment search tool", 1990, J. Mol. Biol, v. 215, pages 403-410.

For multiple sequence analysis, the CLUSTAL W program can be used. The CLUSTAL W program desirably is run using "dynamic" (versus "fast") settings. Amino acid sequences are evaluated using a variable set of BLOSUM matrixes depending on the level of identity between the sequences. The CLUSTAL W program and underlying principles of operation are further described in, e.g., Higgins et al., "CLUSTAL V: improved software for multiple sequence alignment", 1992, CABIOS, 8(2), pages 189-191.

In another embodiment of the first aspect of the invention, optionally in combination with any of the embodiments provided above or below, the peptide or salt thereof is a peptide which has an identity of 85%, 86%, 87%, 88%, 89%, 90%, 91%, 92%, 93%, 94%, 95%, 96%, 97%, 98%, 99% or 100% with respect to SEQ ID NO: 1. In another embodiment of the first aspect of the invention, optionally in combination with any of the embodiments provided above or below, the peptide or salt thereof is a peptide which has an identity of 100% with respect to any of the sequence SEQ ID NO: 1. In one embodiment, optionally in combination with any of the embodiments provided above or below, the two aminoacidic residues connected by the linker are not considered when performing the alignment to determine the identical positions for confirming the identity degree. In one embodiment of the first aspect of the invention the peptide or salt thereof is one consisting of sequence SEQ ID NO:1 as defined above, optionally comprising a "L" biradical of formula (I).

In another embodiment of the first aspect of the invention, optionally in combination with any of the embodiments provided above or below, the peptide or salt thereof is a peptide which has an identity of 85%, 86%, 87%, 88%, 89%, 90%, 91%, 92%, 93%, 94%, 95%, 96%, 97%, 98%, 99% or 100% with respect to SEQ ID NO: 22. In another embodiment of the first aspect of the invention, optionally in combination with any of the embodiments provided above or below, the peptide or salt thereof is a peptide which has an identity of 100% with respect to any of the sequence SEQ ID NO: 22. In one embodiment, optionally in combination with any of the embodiments provided above or below, the two aminoacidic residues connected by the linker are not considered when performing the alignment to determine the identical positions for confirming the identity degree.

As used herein, the term "pharmaceutical acceptable salt" refers to those salts which are, within the scope of sound medical judgment, suitable for use in contact with the tissues of humans and lower animals without undue toxicity, irritation, allergic response and the like, and are commensurate with a reasonable benefit/risk ratio. Pharmaceutical acceptable salts are well known in the art. Examples of pharmaceutical acceptable, nontoxic acid addition salts are salts of an amino group formed with inorganic acids such as hydrochloric acid, hydrobromic acid, phosphoric acid, sulfuric acid and perchloric acid or with organic acids such as acetic acid, trifluoroacetic acid, oxalic acid, maleic acid, tartaric acid, citric acid, succinic acid or malonic acid or by using other methods used in the art such as ion exchange. Other pharmaceutical acceptable salts include adipate, alginate, ascorbate, aspartate, benzenesulfonate, benzoate, bisulphate, borate, butyrate, camphorate, camphorsulfonate, citrate, cyclopentanepropionate, digluconate, dodecylsulphate, ethanesulfonate, formate, fumarate, glucoheptonate, glycerophosphate, gluconate, hemisulphate, heptanoate, hexanoate, hydroiodide, 2-hydroxy-ethanesulfonate, lactobionate, lactate, laurate, lauryl sulphate, malate, maleate, malonate, methanesulfonate, 2-naphthalenesulfonate, nicotinate, nitrate, oleate, oxalate, palmitate, pamoate, pectinate, persulphate, 3-phenylpropionate, phosphate, picrate, pivalate, propionate, stearate, succinate, sulphate, tartrate, thiocyanate, p-toluenesulfonate, undecanoate, valerate salts, and the like. Salts derived from appropriate bases include alkali metal, alkaline earth metal, and ammonium. Representative alkali or alkaline earth metal salts include sodium, lithium, potassium, calcium, magnesium, and the like. Further pharmaceutical acceptable salts include, when appropriate, nontoxic ammonium, quaternary ammonium, and amine cations formed using counterions such as halide, hydroxide, carboxylate, sulphate, phosphate, nitrate, lower alkyl sulfonate and aryl sulfonate.

The term (C₁-C₁₀)alkyl refers to a saturated straight or branched alkyl chain having from 1 to 10 carbon atoms.

Illustrative non-limitative examples are: methyl, ethyl, propyl, isopropyl, butyl, isobutyl, sec-butyl, tert-butyl, n-pentyl, neo-pentyl and n-hexyl.

The term (C₁-C₂₀)alkyl refers to a saturated straight or branched alkyl chain having from 1 to 20 carbon atoms.

The term (C₂-C₁₀)alkenyl refers to a saturated straight, or branched alkyl chain containing from 2 to 10 carbon atoms and also containing one or more double bonds. Illustrative non-limitative examples are ethenyl, propenyl, butenyl, 1-methyl-2-buten-1-yl, and the like.

The term (C₂-C₁₀)alkynyl refers to a saturated straight, or branched alkyl chain containing from 2 to 20 carbon atoms and also containing one or more triple bonds. Examples include, among others, ethynyl, 1-propynyl, 2-butynyl, 1,3-butadinyl, 4-pentynyl, and 1-hexynyl.

The term "halogen" refers to the group in the periodic table consisting of five chemically related elements: fluorine (F), chlorine (CI), bromine (Br), iodine (I), and astatine (At).

The term (C₁-C₁₀)haloalkyl refers to a group resulting from the replacement of one or more hydrogen atoms from a (C₁-C₁₀)alkyl group with one or more, preferably from 1 to 6, halogen atoms, which can be the same or different. Examples include, among others, trifluoromethyl, fluoromethyl, 1-chloroethyl, 2-chloroethyl, 1-fluoroethyl, 2-fluoroethyl, 2-bromoethyl, 2-iodoethyl, 2,2,2-trifluoroethyl, pentafluoroethyl, 3-fluoropropyl, 3-chloropropyl, 2,2,3,3-tetrafluoropropyl, 2,2,3,3,3-pentafluoropropyl, heptafluoropropyl, 4-fluorobutyl, and nonafluorobutyl.

The term "known" ring system as used herein refers to a ring system which is chemically feasible and is known in the art and so intends to exclude those ring systems that are not chemically possible.

According to the present invention when the ring system is formed by "isolated" rings means that the ring system is formed by two, three or four rings and said rings are bound via a bond from the atom of one ring to the atom of the other ring. The term "isolated" also embraces the embodiment in which the ring system has only one ring. Illustrative non-limitative examples of known ring systems consisting of one ring are those derived from: cyclopropyl, cyclobutyl, cyclopentyl, cyclohexyl, cycloheptyl, cyclopropenyl, cyclobutenyl, cyclopentenyl, phenyl, and cycloheptenyl.

According to the present invention when the ring system has rings "totally fused", means that the ring system is formed by two, three or four rings in which two or more atoms are common to two adjoining rings. Illustrative non-limitative examples are 1,2,3,4-tetrahydronaphthyl, 1-naphthyl, 2-naphthyl, anthryl, or phenanthryl.

According to the present invention when the ring system is "partially fused" it means that the ring system is formed by three or four rings, being at least two of said rings totally fused (i.e. two or more atoms being common to the two adjoining rings) and the remaining ring(s) being bound via a bond from the atom of one ring to the atom of one of the fused rings.

Unless otherwise stated, the one or more of the amino acids forming the peptides of the invention can have L- or D-configuration. In one embodiment of the first or second aspect of the invention, optionally in combination with any of the embodiments provided above or below, at least one of the amino acids forming the peptide is a D-amino acid. In another embodiment of the first or second aspect of the invention, optionally in combination with any of the embodiments provided above or below, one of the amino acids forming the peptide is a D-amino acid. In another embodiment of the first or second aspect of the invention, optionally in combination with any of the embodiments provided above or below, at least one of the amino acids connected by the linker is a D-amino acid. In another embodiment of the first or second aspect of the invention, optionally in combination with any of the embodiments provided above or below, one of the amino acids connected by the linker is a D-amino acid and the other a L-amino acid. In another embodiment of the first or second aspect of the invention, optionally in combination with any of the embodiments provided above or below, the amino acids connected by the linker are such that the amino acid closest to the N-terminal is a D-amino acid, whereas the one closest to the C-terminal end is a L-amino acid.

Amino acids used in the construction of peptides of the present invention may be prepared by organic synthesis, or obtained by other routes, such as, for example, degradation of or isolation from a natural source.

In one embodiment of the first aspect of the invention, optionally in combination with any of the embodiments provided above or below, the peptide is one wherein m and n are the same. In another embodiment of the first aspect of the invention, optionally in combination with any of the embodiments provided above or below, the peptide is one wherein p and q are the same. In an alternative embodiment, the peptide is one wherein p and q are different. In another embodiment of the first aspect of the invention, optionally in combination with any of the embodiments provided above or below, m, n, p and q are the same, particularly 1. In another embodiment of the first aspect of the invention, optionally in combination with any of the embodiments provided above or below, m, n and q are the same. In another embodiment of the first aspect of the invention, optionally in combination with any of the embodiments provided above or below, m, n and q represent 0, and p represents 1.

In another embodiment of the first aspect of the invention, optionally in combination with any of the embodiments provided above or below, the peptide is a lineal peptide. In the present invention the term "lineal peptide" means that it does not include the "L" biradical. In another embodiment, optionally in combination with any of the embodiments provided above or below, the lineal peptide is one wherein m, n, p and q are the same. In another embodiment, optionally in combination with any of the embodiments provided above or below, the lineal peptide is one wherein m, n, p and q are the same and represent 1.

In one embodiment of the second aspect of the invention, optionally in combination with any of the embodiments provided above or below, the peptide is one wherein r and s are the same. In another embodiment of the second aspect of the invention, optionally in combination with any of the embodiments provided above or below, the peptide is one wherein t and v are the same. In another embodiment of the second aspect of the invention, optionally in combination with any of the embodiments provided above or below, r and s represent 1, and t and v represent 0. In another embodiment of the second aspect of the invention, optionally in combination with any of the embodiments provided above or below, r and s represent 0, and t and v represent 1.

In another embodiment of the first aspect of the invention, optionally in combination with any of the embodiments provided above or below, the linker biradical of formula (I) is between an alpha carbon atom of an amino acid located at position "i" and an alpha carbon atom of an amino acid located at position "i+7" in the peptide sequence SEQ ID NO: 1.

In another embodiment of the first aspect of the invention, optionally in combination with any of the embodiments provided above or below, the peptide is selected from the group consisting of:
SEQ ID NO: 2 (Leu)m-(Asp)n-Lys-Ala-**Ser**-Val-Met-Arg-Leu-Thr-Ile-**Ser**-Tyr-Leu-Arg-Val-(Arg)p-(Lys)q;
SEQ ID NO: 3 (Leu)m-(Asp)n-Lys-Ala-Ser-Val-**Met**-Arg-Leu-Thr-Ile-Ser-Tyr-**Leu**-Arg-Val-(Arg)p-(Lys)q;
SEQ ID NO: 4: (Leu)m-(Asp)n-Lys-Ala-Ser-Val-Met-**Arg**-Leu-Thr-Ile-Ser-Tyr-Leu-**Arg**-Val-(Arg)p-(Lys)q;
SEQ ID NO: 5 (Leu)m-(Asp)n-Lys-Ala-Ser-Val-Met-Arg-Leu-**Thr**-Ile-Ser-Tyr-Leu-Arg-Val-**Arg**-(Lys)q;
SEQ ID NO: 6: Lys-Ala-**Ser**-Val-Met-Arg-Leu-Thr-Ile-**Ser**-Tyr-Leu-Arg-Val-Arg;
SEQ ID NO: 7: Lys-Ala-Ser-Val-**Met**-Arg-Leu-Thr-Ile-Ser-Tyr-**Leu**-Arg-Val-Arg;
SEQ ID NO: 8: Lys-Ala-Ser-Val-Met-**Arg**-Leu-Thr-Ile-Ser-Tyr-Leu-**Arg**-Val-Arg;
SEQ ID NO: 9: Lys-Ala-Ser-Val-Met-Arg-Leu-**Thr**-Ile-Ser-Tyr-Leu-Arg-Val-**Arg**;
SEQ ID NO: 10: Leu-Asp-Lys-Ala-**Ser**-Val-Met-Arg-Leu-Thr-Ile-**Ser**-Tyr-Leu-Arg-Val-Arg-Lys;
SEQ ID NO: 11: Leu-Asp-Lys-Ala-Ser-Val-**Met**-Arg-Leu-Thr-Ile-Ser-Tyr-**Leu**-Arg-Val-Arg-Lys; and
SEQ ID NO: 12: Leu-Asp-Lys-Ala-Ser-Val-Met-**Arg**-Leu-Thr-Ile-Ser-Tyr-Leu-**Arg**-Val-Arg-Lys;
wherein the bold and underlined amino acids, having the same (being both L-amino acids or D-amino acids) or different configuration, point out the location of the linker biradical "L" (i, i+7), the C-terminal end corresponds to -C(O)R₄, and the N-terminal end corresponds to -NHR₅.

In another embodiment of the second aspect of the invention, optionally in combination with any of the embodiments provided above or below, the linker biradical of formula (I) is between an alpha carbon atom of an amino acid located at position "i" and an alpha carbon atom of an amino acid located at position "i+7" in the peptide sequence SEQ ID NO: 22.

In another embodiment of the second aspect of the invention, optionally in combination with any of the embodiments provided above or below, the peptide is selected from the group consisting of:
SEQ ID NO: 23 (Arg)r-(Ser)s-Arg-Arg-Ser-Lys-Glu-**Ser**-Glu-Val-Phe-Tyr-Glu-Leu-**Ala**-His-Gln-(Leu)t-(Pro)v;
SEQ ID NO: 24 (Arg)r-(Ser)s-Arg-Arg-Ser-Lys-Glu-Ser-Glu-**Val**-Phe-Tyr-Glu-Leu-Ala-His-**Gln**-(Leu)-(Pro)v;
SEQ ID NO: 25: (Arg)r-(Ser)s-Arg-Arg-**Ser**-Lys-Glu-Ser-Glu-Val-Phe-**Tyr**-Glu-Leu-Ala-His-Gln-(Leu)t-(Pro)v;
SEQ ID NO: 26: Arg-Ser-Arg-Arg-Ser-Lys-Glu-**Ser**-Glu-Val-Phe-Tyr-Glu-Leu-**Ala**-His-Gln;
SEQ ID NO: 27: Arg-Ser-Arg-Arg-Ser-Lys-Glu-Ser-Glu-**Val**-Phe-Tyr-Glu-Leu-Ala-His-**Gln**; and
SEQ ID NO: 28: Arg-Arg-**Ser**-Lys-Glu-Ser-Glu-Val-Phe-**Tyr**-Glu-Leu-Ala-His-Gln-Leu-Pro,
wherein the bold and underlined amino acids, having the same (being both L-amino acids or D-amino acids) or different configuration, point out the location of the linker biradical "L" (i, i+7), the C-terminal end corresponds to -C(O)R₄, and the N-terminal end corresponds to -NHR₅.

In another embodiment of the first or second aspect of the invention, optionally in combination with any of the embodiments provided above or below, the peptide or salt thereof is one comprising a "L" linker wherein a= 1. In another embodiment of the first or second aspect of the invention, optionally in combination with any of the embodiments provided above or below, the peptide or salt thereof is one comprising a "L" linker wherein b= 1. In another embodiment of the first aspect of the invention, optionally in combination with any of the embodiments provided above or below, the peptide or salt thereof is one comprising a "L" linker wherein c= 1. In another embodiment of the first aspect of the invention, optionally in combination with any of the embodiments provided above or below, the peptide or salt thereof is one comprising a "L" linker wherein a=b=c= 1.

In another embodiment of the first aspect of the invention, optionally in combination with any of the embodiments provided above or below, the peptide or salt thereof is one comprising a "L" linker wherein R₁ and R₃ are biradicals independently selected from the group consisting of: (C₁-C₁₀)alkyl; (C₂-C₁₀)alkenyl; and (C₂-C₁₀)alkynyl. In another embodiment of the first aspect of the invention, optionally in combination with any of the embodiments provided above or below, the peptide or salt thereof is one comprising a "L" linker wherein R₁ and R₃ are the same or different and represent (C₁-C₁₀)alkyl.

In another embodiment of the first or second aspect of the invention, optionally in combination with any of the embodiments provided above or below, the peptide or salt thereof is one comprising a "L" linker wherein R₂ is a biradical selected from the group consisting: -O-, C(=O), C(=O)NR₁₃, C(=O)O, S(=O), S(=O)₂, NR₁₄, (C₁-C₁₀)alkyl, (C₂-C₁₀)alkenyl, (C₂-C₁₀)alkynyl, -NR₁₅-NR₁₆-, -N=N-, -S-S-, and a known ring system consisting of one ring from 3 to 6 members, the ring:
being saturated, partially unsaturated, or aromatic;
each one of the members forming the known ring system being selected from the group consisting of: -CH-, -CH₂-, -NH-, -N-, -SH-, -S-, and -O-; and
the ring system being optionally substituted by one or more radicals independently selected from the group consisting of halogen, -OH, -NO₂, (C₁-C₁₀)alkyl, (C₁-C₁₀)haloalkyl, and (C₁-C₁₀)alkyl-O-.

In another embodiment of the first or second aspect of the invention, optionally in combination with any of the embodiments provided above or below, the peptide or salt thereof is one comprising a "L" linker wherein R₂ is a biradical selected from the group consisting of: (C₁-C₁₀)alkyl, (C₂-C₁₀)alkenyl, and (C₂-C₁₀)alkynyl. In another embodiment of the first aspect of the invention, optionally in combination with any of the embodiments provided above or below, the peptide or salt thereof is one comprising a "L" linker wherein R₂ is (C₂-C₁₀)alkenyl.

In another embodiment of the first or second aspect of the invention, optionally in combination with any of the embodiments provided above or below, the peptide or salt thereof is one comprising a "L" linker wherein R₁ and R₃ are the same or different and represent (C₁-C₁₀)alkyl; and R₂ is (C₂-C₁₀)alkenyl.

In another embodiment of the first or second aspect of the invention, optionally in combination with any of the embodiments provided above or below, the peptide or salt thereof is one comprising a "L" linker wherein R₁ and R₃ are the same or different and represent (C₁-C₁₀)alkyl; R₂ is (C₂-C₁₀)alkenyl; and a=b=c= 1.

In another embodiment of the first or second aspect of the invention, optionally in combination with any of the embodiments provided above or below, the peptide or salt thereof is one wherein R₁₉ is selected from the group consisting of: (C₁-C₁₀)alkyl, (C₂-C₁₀)alkenyl, and (C₂-C₁₀)alkynyl. In another embodiment of the first or second aspect of the invention, optionally in combination with any of the embodiments provided above or below, the peptide or salt thereof is one wherein R₁₉ is a (C₁-C₁₀)alkyl monoradical. In another embodiment of the first or second aspect of the invention, optionally in combination with any of the embodiments provided above or below, the peptide or salt thereof is one wherein R₁, R₃ and R₁₉ are the same or different and represent (C₁-C₁₀)alkyl; and R₂ is (C₂-C₁₀)alkenyl.

In one embodiment of the first aspect of the invention, optionally in combination with any of the embodiments provided above or below, the peptide is one wherein:
- R₁, R₃ and R₁₉ are (C₁-C₁₀)alkyl; R₂ is (C₂-C₁₀)alkenyl; "m" and "n" are the same; and "p" and "q" are the same; or, alternatively,
- R₁, R₃ and R₁₉ are (C₁-C₁₀)alkyl; R₂ is (C₂-C₁₀)alkenyl; "m" and "n" are the same; and "p" and "q" are different; or, alternatively,
- R₁, R₃ and R₁₉ are (C₁-C₁₀)alkyl; R₂ is (C₂-C₁₀)alkenyl; "m", "n", "p" and "q" are the same, particularly are the same and represent 1; or, alternatively,
- R₁, R₃ and R₁₉ are (C₁-C₁₀)alkyl; R₂ is (C₂-C₁₀)alkenyl; "m", "n", "q" are the same and "q" is different; particularly, m=n=q= 0 and q=1.

In one embodiment of the second aspect of the invention, optionally in combination with any of the embodiments provided above or below, the peptide is one wherein:
- R₁, R₃ and R₁₉ are (C₁-C₁₀)alkyl; R₂ is (C₂-C₁₀)alkenyl; "r" and "s" are the same; and "t" and "v" are the same; or alternatively,
- R₁, R₃ and R₁₉ are (C₁-C₁₀)alkyl; R₂ is (C₂-C₁₀)alkenyl; "r" and "s" are the same and represent 1; and "t" and "v" are the same and represent 0; or alternatively
- R₁, R₃ and R₁₉ are (C₁-C₁₀)alkyl; R₂ is (C₂-C₁₀)alkenyl; "r" and "s" are the same and represent 0; and "t" and "v" are the same and represent 1.

In another embodiment of the first or second aspect of the invention, optionally in combination with any of the embodiments provided above or below, the peptide or salt thereof is one wherein R₄ is -OH (i.e., the C-terminal end is -C(O)OH). In another embodiment of the first or second aspect of the invention, optionally in combination with any of the embodiments provided above or below, the peptide or salt thereof is one wherein R₄ is -NR₁₇R₁₈, R₁₇ and R₁₈ meaning the same. In another embodiment of the first or second aspect of the invention, optionally in combination with any of the embodiments provided above or below, the N-terminal end corresponds to -NH₂. In another embodiment of the first or second aspect of the invention, optionally in combination with any of the embodiments provided above or below, the C-terminal and N-terminal ends of the peptide of the invention are, respectively, -C(O)OH and -NH₂. In another embodiment of the first or second aspect of the invention, optionally in combination with any of the embodiments provided above or below, the C-terminal and N-terminal ends of the peptide of the invention are, respectively, -C(O)NH₂ and -NH₂.

In another embodiment of the first or second aspect of the invention, optionally in combination with any of the embodiments provided above or below, the C-terminal and N-terminal ends of the peptide of the invention are, respectively, -C(O)R₄ and -NH₂, wherein R₄ means -NHR₁₈, and R₁₈ is a radical selected from the group consisting of: (C₁-C₁₀)alkyl and (C₁-C₁₀)alkyl substituted by one or more radicals selected from the group consisting of: halogen, (C₁-C₁₀)alkyl, -OR₆, -NR₇R₈, -SR₉, -SOR₁₀, -SO₂R₁₁, and -CO₂R₁₂.

In another embodiment of the first or second aspect of the invention, optionally in combination with any of the embodiments provided above or below, the C-terminal and N-terminal ends of the peptide of the invention are, respectively, -C(O)R₄ and -NH₂, wherein R₄ means -NR₁₇R₁₈, and R₁₇ and R₁₈ are radicals independently selected from the group consisting of: (C₁-C₁₀)alkyl and (C₁-C₁₀)alkyl substituted by one or more radicals selected from the group consisting of: halogen, (C₁-C₁₀)alkyl, -OR₆, -NR₇R₈, -SR₉, -SOR₁₀, -SO₂R₁₁, and -CO₂R₁₂.

In another embodiment of the first or second aspect of the invention, optionally in combination with any of the embodiments provided above or below, the C-terminal and N-terminal ends of the peptide of the invention are, respectively, -C(O)OH and -NHR₅, wherein R₅ is selected from (C₁-C₂₀)alkyl and (C₁-C₁₀)alkyl substituted by one or more radicals selected from the group consisting of: halogen, (C₁-C₁₀)alkyl, -OR₆, -NR₇R₈, -SR₉, - SOR₁₀, -SO₂R₁₁, and -CO₂R₁₂.

In another embodiment of the first and second aspect of the invention, optionally in combination with any of the embodiments provided above or below, the peptide or salt thereof is one wherein R₁, R₃ and R₁₉ are the same or different and represent (C₁-C₁₀)alkyl; and R₂ is (C₂-C₁₀)alkenyl. In another embodiment of the first and second aspects of the invention, optionally in combination with any of the embodiments provided above or below, the peptide or salt thereof is one, wherein R₁, R₃ and R₁₉ are the same or different and represent (C₁-C₁₀)alkyl; R₂ is (C₂-C₁₀)alkenyl; the C-terminal is selected from -C(O)OH; and -CONH₂ and the N-terminal end is -NH₂.

In another embodiment of the first aspect of the invention, the peptide is one having at least a 85, 86, 87, 88, 89, 90, 91, 92, 93, 94, 95, 96, 97, 98, 99 or a 100% of identity with respect to a sequence selected from the group consisting of sequence SEQ ID NO: 13 to 20:
SEQ ID NO: 13
Leu-Asp-Lys-Ala-Ser-Val-Met-Arg-Leu-Thr-Ile-Ser-Tyr-Leu-Arg-Val-Arg-Lys
wherein the N-terminal end corresponding to -NH₂, and a C-terminal end corresponding to -C(O)NH_{2.} 
SEQ ID NO: 14
(CH2)6 CH=CH (CH2)3
Lys-Ala-NH-C-C(O)-Val-Met-Arg-Leu-Thr-Ile-NH-C-C(O)-Tyr-Leu-Arg-Val-Arg
CH3 CH3
 wherein the N-terminal end corresponding to -NH₂, the C-terminal end corresponding to -C(O)NH_{2.}, the amino acid at position 3 being a D-amino acid, and the remaining amino acids being L-amino acids. 
SEQ ID NO: 15

(CH2)6 CH=CH (CH2)3

Lys-Ala-Ser-Val-NH-C-C(O)-Arg-Leu-Thr-Ile-Ser-Tyr-NH-C-C(O)-Arg-Val-Arg

CH3 CH3
 wherein the N-terminal end corresponding to -NH₂, a C-terminal end corresponding to -C(O)NH_{2,} the amino acid at position 5 being a D-amino acid, and the remaining amino acids being L-amino acids. 
SEQ ID NO: 16

(CH2)6 CH=CH (CH2)3

Lys-Ala-Ser-Val-Met-NH-C-C(O)-Leu-Thr-Ile-Ser-Tyr-Leu-NH-C-C(O)-Val-Arg

CH3 CH3
 wherein the N-terminal end corresponding to -NH₂, a C-terminal end corresponding to -C(O)NH_{2,} the amino acid at position 6 being a D-amino acid, and the remaining amino acids being L-amino acids. 
SEQ ID NO: 17
(CH2)6 CH=CH (CH2)3
Lys-Ala-Ser-Val-Met-NH-C-C(O)-Leu-Thr-Ile-Ser-Tyr-Leu-NH-C-C(O)-Val-Arg
CH3 CH3
 wherein the N-terminal end corresponding to -NH₂, a C-terminal end corresponding to -C(O)OH, the amino acid at position 6 being a D-amino acid, and the remaining amino acids being L-amino acids. 
SEQ ID NO: 18
(CH2)6 CH=CH (CH2)3
Lys-Ala-Ser-Val-Met-Arg-Leu-NH-C-C(O)-Ile-Ser-Tyr-Leu-Arg-Val-NH-C-C(O)NH2
CH3 CH3
 wherein the N-terminal end corresponding to -NH₂, a C-terminal end corresponding to -C(O)NH_{2,} the amino acid at position 8 being a D-amino acid, and the remaining amino acids being L-amino acids. 
SEQ ID NO: 19

(CH2)6 CH=CH (CH2)3

Leu-Asp-Lys-Ala-NH-C-C(O)-Val-Met-Arg-Leu-Thr-Ile-NH-C-C(O)-Tyr-Leu-Arg-Val-Arg-Lys

CH3 CH3
 wherein the N-terminal end corresponding to -NH₂ ,a C-terminal end corresponding to -C(O)NH_{2,} the amino acid at position 5 being a D-amino acid, and the remaining amino acids being L-amino acids. 
SEQ ID NO: 20

(CH2)6 CH=CH (CH2)3

Leu-Asp-Lys-Ala-Ser-Val-NH-C-C(O)-Arg-Leu-Thr-Ile-Ser-Tyr-NH-C-C(O)-Arg-Val-Arg-Lys

CH3 CH3
 wherein the N-terminal end corresponding to -NH₂, a C-terminal end corresponding to -C(O)NH_{2.}, the amino acid at position 7 being a D-amino acid, and the remaining amino acids being L-amino acids. 
SEQ ID NO: 21
(CH2)6 CH=CH (CH2)3
Leu-Asp-Lys-Ala-Ser-Val-Met-NH-C-C(O)-Leu-Thr-Ile-Ser-Tyr-Leu-NH-C-C(O)-Val-Arg-Lys
CH3 CH3
 wherein the N-terminal end corresponding to -NH₂, a C-terminal end corresponding to -C(O)NH_{2.}, the amino acid at position 8 being a D-amino acid, and the remaining amino acids being L-amino acids.

In another embodiment of the first aspect of the invention, the peptide is one selected from the group consisting of sequence SEQ ID NO: 13 to 20.

In another embodiment of the second aspect of the invention, the peptide is one having a sequence identity of at least 85, 86, 87, 88, 89, 90, 91, 92, 93, 94, 95, 96, 97, 98, 99 or of 100% with respect to a sequence selected from the group consisting of SEQ ID NO: 29 to 31: 
SEQ ID NO: 29
(CH2)6 CH=CH (CH2)3
Arg-Ser-Arg-Arg-Ser-Lys-Glu-NH-C-C(O)-Glu-Val-Phe-Tyr-Glu-Leu-NH-C-C(O)-Hys-Gln
CH3 CH3
 wherein the N-terminal end corresponding to -NH₂, a C-terminal end corresponding to -C(O)NH_{2,} the amino acid at position 8 being a D-amino acid, and the remaining amino acids being L-amino acids. 
SEQ ID NO: 30

(CH2)6 CH=CH (CH2)3

Arg-Ser-Arg-Arg-Ser-Lys-Glu-Ser-Glu-NH-C-C(O)-Phe-Tyr-Glu-Leu-Ala-Hys-NH-C-C(O)-NH2

CH3 CH3
 wherein the N-terminal end corresponding to -NH₂, a C-terminal end corresponding to -C(O)NH_{2.}, the amino acid at position 10 being a D-amino acid, and the remaining amino acids being L-amino acids. 
SEQ ID NO: 31

(CH2)6 CH=CH (CH2)3

Arg-Arg-NH-C-C(O)-Lys-Glu-Ser-Glu-Val-Phe-NH-C-C(O)-Glu-Leu-Ala-Hys-Gln-Leu-Pro

CH3 CH3
 wherein the N-terminal end corresponding to -NH₂, a C-terminal end corresponding to -C(O)NH_{2,} the amino acid at position 3 being a D-amino acid, and the remaining amino acids being L-amino acids.

In another embodiment of the second aspect of the invention, optionally in combination with any of the embodiments provided above, the peptide is selected from the group consisting of SEQ ID NO: 29 to 31.

The process for the preparation of the peptides according to the first aspect of the invention comprises:
(1.a) the coupling, by condensation, of the corresponding amino acids of the peptide with a compound of formula (III) and a compound of formula (IV), which correspond to the amino acids referred as "i" and "i+4" or "i+7". Compounds (III) and (IV) will be those suffering a subsequent cyclization step to generate the "L" biradical": 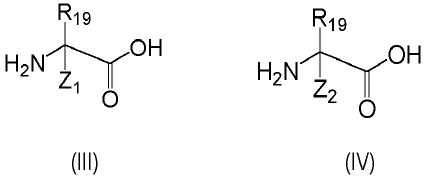 wherein R₁₉ is as defined above, Z₁ and Z₂ are the same or different and represent (C₂-C₁₀)alkenyl; and
(1.b) a cyclization step comprising the ring-closed metathesis of Z₁ and Z₂ (cf. Kim Young-Woo et al., "Synthesis of all-hydrocarbon stapled a-helical peptides by ring-closing olefin metathesis", Nature Protocols, 2011, 6(6), p. 761-771; Scott J. M. et al., "Application of Ring-Closing Metathesis to the Synthesis of Rigidified Amino Acids and Peptides", J. Am. Chem. Soc., 1996, v.118 (40), pp 9606-9614) performed in solution with a Grubbs (I or II generation) catalyst; or, alternatively,
(2a) the coupling, by condensation, of the required amino acids, including a compound of formula (V) and a compound of formula (VI), which correspond to the amino acids referred as "i" and "i+4" or "i+7". Compounds (V) and (VI) will be those suffering a subsequent cyclization step to generate the "L" biradical": 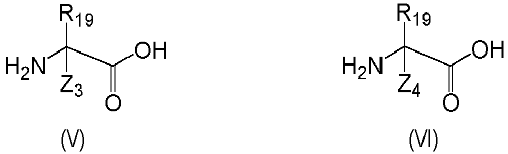 wherein R₁₉ is as defined above, Z₃ and Z₄ are the same or different, selected from the group consisting of: halogen -SH, -NHR₂₀, -OH, (C₂-C₁₀)alkyl-SH, (C₁-C₁₀)alkyl-OH, (C₁-C₁₀)alkyl-NHR₂₁, C(=O)OH, (C₁-C₁₀)C(=O)OH, C(=O)NHR₂₂, (C₁-C₁₀)alkyl-C(=O)NHR₂₃, OR₂₄, C(=O)-halogen, C(=O)-OR₂₅, S(=O)-halogen, S(=O)-OR₂₆, S(=O)₂R₂₇ where R₂₀, R₂₁ R₂₂, R₂₃, R₂₄, R₂₅ R₂₆ and R₂₇ are monoradicals selected from the group consisting of: hydrogen, (C₁-C₁₀)alkyl, (C₂-C₁₀)alkenyl, and (C₂-C₁₀)alkynyl; a known ring system comprising from 3 to 14 carbon atoms, the system comprising from 1 to 3 rings, where:
   each one of the rings is saturated, partially unsaturated, or aromatic;
   the rings are isolated, partially or totally fused,
   each one of the members forming the known ring system is selected from the group consisting of: -CH-, -CH₂-, -NH-, -N-, -SH-, -S-, and -O-; and
   the ring system is optionally substituted by one or more radicals independently selected from the group consisting of halogen, -OH, -NH₂, -SH, C(=O)-halogen (C₁-C₁₀)haloalkyl, and (C₁-C₁₀)alkyl-O-; and
(2b) a cyclization step comprising the coupling reaction between Z₃ and Z₄ radicals; or, alternatively,
(3a) the coupling, by condensation, of the corresponding amino acids of the peptide with a compound of formula (VII) and a compound of formula (VIII), which correspond to the amino acids referred as "i" and "i+4" or "i+7". Compounds (VII) and (VIII) will be those suffering a subsequent cyclization step to generate the "L" biradical": 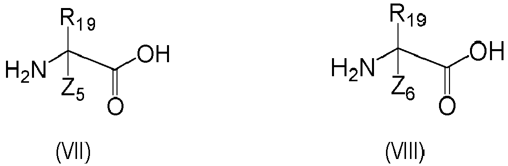 wherein R₁₉ is as defined above, one of Z₅ and Z₆ is (C₂-C₁₀)alkynyl and the other is (C₂-C₁₀)alkylN₃; and
(3.b) a cyclization step comprising the condensation of Z₅ and Z₆ radicals by well-known protocols such as the Cu(I)-mediated Huisgen 1,3-dipolar cycloaddition reaction (a.k.a. a "click" reaction) to generate a 1,4-substituted 1,2,3-triazole bridge (cf. Kolb H.C. et al., "The growing impact of click chemistry on drug discovery", 2003, Drug Discov Today, 8(24):1128-1137).

The process for the preparation of the peptide according to the second aspect of the invention comprises the coupling, by condensation, of the carboxylic group or C-terminus of one amino acid with the amino group or N-terminus of another, this coupling reaction being repeated the number of times required until the desired peptide is obtained.

The compounds of formula (III), (IV), (V), (VI), (VII), and (VIII) are commercially available and are coupled by condensation to the already formed portion of peptide sequence. These compounds can carry beads for the appropriate solid phase synthesis of the peptide, as well as protecting groups of the carboxy, amino or side-chain. Illustrative non-limitative examples of compounds are: 2-(2'-propenyl)alanine, 2-(3'-butenyl)glycine, 2-(4'-pentenyl)alanine, 2-(6'-heptenyl) alanine, 2-(7'-octenyl)alanine, allyl-glycine, 5-azido-norvaline, and alpha-propargyl-alanine, among others.

The "coupling" step can be performed in solid phase, following the protocol "deprotection-wash-coupling-wash", by condensation of the carboxylic group of one amino acid with the amino group of another amino acid residue, using amino acids as defined above as well as alpha-alpha di-substituted amino acids of formula (III) to (VIII).

The general principle of solid phase peptide synthesis is to repeat cycles of deprotection-wash-coupling-wash. The free N-terminal amine of a solid-phase attached peptide is coupled to a single N-protected amino acid unit. This unit is then deprotected, revealing a new N-terminal amine to which a further amino acid may be attached. Amino acids have reactive moieties at the N- and C-termini, which facilitates amino acid coupling during synthesis. Many amino acids also have reactive side chain functional groups, which can interact with free termini or other side chain groups during synthesis and peptide elongation and negatively influence yield and purity. To facilitate proper amino acid synthesis with minimal side chain reactivity, chemical groups have been developed to bind to specific amino acid functional groups and block, or protect, the functional group from nonspecific reactions. These protecting groups, while vast in nature, can be separated into three groups, as follows: N-terminal protecting groups, C-terminal protecting groups (mostly used in liquid-phase synthesis), and side chain protecting groups.

For coupling the peptides the carboxyl group is usually activated. This is important for speeding up the reaction. There are two main types of activating groups: carbodiimides and triazolols. However, the use of pentafluorophenyl esters (FDPP, PFPOH]) and BOP-CI are useful for cyclising peptides.

Purified, individual amino acids are reacted with these protecting groups prior to synthesis and then selectively removed during specific steps of peptide synthesis.

Exemplary resins which may be employed by the present invention include, but are not limited to: (1) alkenyl resins (e.g., REM resin, vinyl sulfone polymer-bound resin, vinyl-polystyrene resin); (2) amine functionalized resins (e.g., amidine resin, N-(4-Benzyloxybenzyl)hydroxylamine polymer bound, (aminomethyl)polystyrene, polymer bound (R)-(+)-a-methylbenzylamine, 2-Chlorotrityl Knorr resin, 2-N-Fmoc-Amino-dibenzocyclohepta-1,4-diene, polymer-bound resin, 4-[4-(1-Fmoc-aminoethyl)-2-methoxy-5-nitrophenoxy]butyramidomethyl-polystyrene resin, 4-Benzyloxybenzylamine, polymer-bound, 4-Carboxybenzenesulfonamide, polymer-bound, Bis(tert-butoxycarbonyl)thiopseudourea, polymer-bound, Dimethylaminomethyl-polystyrene, Fmoc-3-amino-3-(2-nitrophenyl)propionic acid, polymer-bound, N-Methyl aminomethylated polystyrene, PAL resin, Sieber amide resin, tert-Butyl N-(2-mercaptoethyl)carbamate, polymer-bound, Triphenylchloromethane-4-carboxamide polymer bound); (3) benzhydrylamine (BHA) resins (e.g., 2-Chlorobenzhydryl chloride, polymer-bound, HMPB-benzhydrylamine polymer bound, 4-Methylbenzhydrol, polymer-bound, Benzhydryl chloride, polymer-bound, Benzhydrylamine polymer-bound); (4) Br-functionalized resins (e.g., 4-(Benzyloxy)benzyl bromide polymer bound, 4-Bromopolystyrene, Brominated PPOA resin, Brominated Wang resin, Bromoacetal, polymer-bound, Bromopolystyrene, HypoGel^{®} 200 Br, Polystyrene A-Br for peptide synthesis, Selenium bromide, polymer-bound, TentaGel HL-Br, TentaGel MB-Br, TentaGel S-Br); (5) Chloromethyl resins (e.g., 5-[4-(Chloromethyl)phenyl]pentyl]styrene, polymer-bound, 4-(Benzyloxy)benzyl chloride polymer bound, 4-Methoxybenzhydryl chloride, polymer-bound); (6) CHO-functionalized resins (e.g., (4-Formyl-3-methoxyphenoxymethyl)polystyrene, (4-Formyl-3-methoxyphenoxymethyl)polystyrene, 3-Benzyloxybenzaldehyde, polymer-bound, 4-Benzyloxy-2,6-dimethoxybenzaldehyde, polymer-bound, Formylpolystyrene, HypoGel^{®} 200 CHO, Indole resin, Polystyrene A-CH(OEt)2, TentaGel HL-CH(OEt)2); (7) Cl-functionalized resins (e.g., Benzoyl chloride polymer bound, (chloromethyl)polystyrene, Merrifield's resin); (8) CO2H functionalized resins (e.g., Carboxyethylpolystryrene, HypoGel^{®} 200 COOH, Polystyrene AM-COOH, TentaGel HL-COOH, TentaGel MB-COOH, TentaGel S-COOH); (9) Hypo-Gel resins (e.g., HypoGel^{®} 200 FMP, HypoGel^{®} 200 PHB, HypoGel^{®} 200 Trt-OH, HypoGel^{®} 200 HMB); (10) I-functionalized resins (e.g., 4-lodophenol, polymer-bound, lodopolystyrene); Janda-Jels^{™} (JandaJel<ä>-Rink amide, JandaJel-NH2, JandaJel-Cl, JandaJel-4-Mercaptophenol, JandaJel-OH, JandaJel-1-(3-Dimethylaminopropyl)-3-ethylcarbodiimide, JandaJel-1,3,4,6,7,8-hexahydro-2H-pyrimido-[1,2-a]pyrimidine, JandaJel-morpholine, JandaJel-polypyridine, JandaJel-Triphenylphosphine, JandaJel-Wang); (11) MBHA resins (3[4'-(Hydroxymethyl)phenoxy]propionic acid-4-methylbenzhydrylamine resin, 4-(Hydroxymethyl)phenoxyacetic acid polymer-bound to MBHA resin, HMBA-4-methylbenzhydrylamine polymer bound, 4-Methylbenzhydrylamine hydrochloride polymer bound Capacity (amine)); (12) NH2 functionalized resins ((Aminomethyl)polystyrene, (Aminomethyl)polystyrene, HypoGel^{®} 200 NH2, Polystyrene AM-NH2, Polystyrene Microspheres 2-aminoethylated, Polystyrol Microspheres 2-bromoethylated, Polystyrol Microspheres 2-hydroxyethylated, TentaGel HL-NH2, Tentagel M Br, Tentagel M NH2, Tentagel M OH, TentaGel MB-NH2, TentaGel S-NH2, TentaGel S-NH2); (13) OH-functionalized resins (e.g., 4-hydroxymethylbenzoic acid, polymer-bound, Hydroxymethyl Resins, OH-functionalized Wang Resins); (14) oxime resins (e.g., 4-Chlorobenzophenone oxime polymer bound, Benzophenone oxime polymer bound, 4-Methoxybenzophenone oxime polymer bound); (15) PEG resins (e.g., ethylene glycol polymer bound); (16) Boc-/Blz peptide synthesis resins (e.g., Boc-Lys(Boc)-Lys[Boc-Lys(Boc)]-Cys(Acm)-b-Ala-O-PAM resin, Boc-Lys(Fmoc)-Lys[Boc-Lys(Fmoc)]-b-Ala-O-Pam resin, Boc-Lys(Boc)-Lys[Boc-Lys(Boc)]-Lys{Boc-Lys(Boc)-Lys[Boc-Lys(Boc)]}-b-Ala-O-PAM resin, Boc-Lys(Fmoc)-Lys[Boc-Lys(Fmoc)]-Lys[Boc-Lys(Fmoc)-Lys{Boc-Lys(Fmoc)]}-b-Ala-O-PAM resin, Boc-Lys(Boc)-Lys[Boc-Lys(Boc)]-Lys{Boc-Lys(Boc)-Lys[Boc-Lys(Boc)]}-Cys(Acm)-b-Ala-O-PAM resin, Preloaded PAM resins); (17) Fmoc-/t-Bu peptide synthesis resins (e.g., Fmoc-Lys(Fmoc)-Lys[Fmoc-Lys(Fmoc)]-b-Ala-O-Wang resin, Fmoc-Lys(Fmoc)-Lys[Fmoc-Lys(Fmoc)]-Lys{Fmoc-Lys(Fmoc)-Lys[Fmoc-Lys(Fmoc)]}-b-Ala-O-Wang resin, Preloaded TentaGel^{®} S Trityl Resins, Preloaded TentaGel^{®} Resins, Preloaded Trityl Resins, Preloaded Wang Resins, Trityl Resins Preloaded with Amino Alcohols); (19) thiol-functionalized resins (e.g., HypoGel^{®} 200 S-Trt, Polystyrene AM-S-Trityl, TentaGel HL-S-Trityl, TentaGel MB-S-Trityl, TentaGel S-S-Trityl); and (20) Wang resins (e.g., Fmoc-Ala-Wang resin, Fmoc-Arg(Pbf)-Wang resin, Fmoc-Arg(Pmc)-Wang resin, Fmoc-Asn(Trt)-Wang resin, Fmoc-Asp(OtBu)-Wang resin, Fmoc-Cys(Acm)-Wang resin, Fmoc-Cys(StBu)-Wang resin, Fmoc-Cys(Trt) Wang resin, Fmoc-Gln(Trt)-Wang resin, Fmoc-Glu(OtBu)-Wang resin, Fmoc-Gly-Wang resin, Fmoc-His(Trt)-Wang resin, Fmoc-Ile-Wang resin, Fmoc-Leu-Wang resin, Fmoc-Lys(Boc)-Wang resin, Fmoc-Met-Wang resin, Fmoc-D-Met-Wang resin, Fmoc-Phe-Wang resin, Fmoc-Pro-Wang resin, Fmoc-Ser(tBu)-Wang resin, Fmoc-Ser(Trt)-Wang resin, Fmoc-Thr(tBu)-Wang resin, Fmoc-Trp(Boc) Wang resin, Fmoc-Trp-Wang resin, Fmoc-Tyr(tBu)-Wang resin, Fmoc-Val-Wang resin).

"Protecting group" (PG) refers to a grouping of atoms that when attached to a reactive group in a molecule masks, reduces or prevents that reactivity.

Suitable amino-protecting groups include methyl carbamate, ethyl carbamante, 9-fluorenylmethyl carbamate (Fmoc), 9-(2-sulfo)fluorenylmethyl carbamate, 9-(2,7-dibromo)fluoroenylmethyl carbamate, 2,7-di-t-butyl-[9-(10,10-dioxo-10,10,10,10-tetrahydrothioxanthyl)]methyl carbamate (DBD-Tmoc), 4-methoxyphenacyl carbamate (Phenoc), 2,2,2-trichloroethyl carbamate (Troc), 2-trimethylsilylethyl carbamate (Teoc), 2-phenylethyl carbamate (hZ), 1-(1-adamantyl)-1-methylethyl carbamate (Adpoc), 1,1-dimethyl-2-haloethyl carbamate, 1,1-dimethyl-2,2-dibromoethyl carbamate (DB-t-BOC), 1,1-dimethyl-2,2,2-trichloroethyl carbamate (TCBOC), 1-methyl-1-(4-biphenylyl)ethyl carbamate (Bpoc), 1-(3,5-di-t-butylphenyl)-1-methylethyl carbamate (t-Bumeoc), 2-(2'- and 4'-pyridyl)ethyl carbamate (Pyoc), 2-(N,N-dicyclohexylcarboxamido)ethyl carbamate, t-butyl carbamate (BOC), 1-adamantyl carbamate (Adoc), vinyl carbamate (Voc), allyl carbamate (Alloc), 1-isopropylallyl carbamate (Ipaoc), cinnamyl carbamate (Coc), 4-nitrocinnamyl carbamate (Noc), 8-quinolyl carbamate, N-hydroxypiperidinyl carbamate, alkyldithio carbamate, benzyl carbamate (Cbz), p-methoxybenzyl carbamate (Moz), p-nitobenzyl carbamate, p-bromobenzyl carbamate, p-chlorobenzyl carbamate, 2,4-dichlorobenzyl carbamate, 4-methylsulfinylbenzyl carbamate (Msz), 9-anthrylmethyl carbamate, diphenylmethyl carbamate, 2-methylthioethyl carbamate, 2-methylsulfonylethyl carbamate, 2-(p-toluenesulfonyl)ethyl carbamate, [2-(1,3-dithianyl)]methyl carbamate (Dmoc), 4-methylthiophenyl carbamate (Mtpc), 2,4-dimethylthiophenyl carbamate (Bmpc), 2-phosphonioethyl carbamate (Peoc), 2-triphenylphosphonioisopropyl carbamate (Ppoc), 1,1-dimethyl-2-cyanoethyl carbamate, m-chloro-p-acyloxybenzyl carbamate, p-(dihydroxyboryl)benzyl carbamate, 5-benzisoxazolylmethyl carbamate, 2-(trifluoromethyl)-6-chromonylmethyl carbamate (Tcroc), m-nitrophenyl carbamate, 3,5-dimethoxybenzyl carbamate, o-nitrobenzyl carbamate, 3,4-dimethoxy-6-nitrobenzyl carbamate, phenyl(o-nitrophenyl)methyl carbamate, phenothiazinyl-(10)-carbonyl derivative, N'-p-toluenesulfonylaminocarbonyl derivative, N'-phenylaminothiocarbonyl derivative, t-amyl carbamate, S-benzyl thiocarbamate, p-cyanobenzyl carbamate, cyclobutyl carbamate, cyclohexyl carbamate, cyclopentyl carbamate, cyclopropylmethyl carbamate, p-decyloxybenzyl carbamate, 2,2-dimethoxycarbonylvinyl carbamate, o-(N,N-dimethylcarboxamido)benzyl carbamate, 1,1-dimethyl-3-(N,N-dimethylcarboxamido)propyl carbamate, 1,1-dimethylpropynyl carbamate, di(2-pyridyl)methyl carbamate, 2-furanylmethyl carbamate, 2-iodoethyl carbamate, isobornyl carbamate, isobutyl carbamate, isonicotinyl carbamate, p-(p'-methoxyphenylazo)benzyl carbamate, 1-methylcyclobutyl carbamate, 1-methylcyclohexyl carbamate, 1-methyl-1-cyclopropylmethyl carbamate, 1-methyl-1-(3,5-dimethoxyphenyl)ethyl carbamate, 1-methyl-1-(p-phenylazophenyl)ethyl carbamate, 1-methyl-1-phenylethyl carbamate, 1-methyl-1-(4-pyridyl)ethyl carbamate, phenyl carbamate, p-(phenylazo)benzyl carbamate, 2,4,6-tri-t-butylphenyl carbamate, 4-(trimethylammonium)benzyl carbamate, 2,4,6-trimethylbenzyl carbamate, formamide, acetamide, chloroacetamide, trichloroacetamide, trifluoroacetamide, phenylacetamide, 3-phenylpropanamide, picolinamide, 3-pyridylcarboxamide, N-benzoylphenylalanyl derivative, benzamide, p-phenylbenzamide, o-nitophenylacetamide, o-nitrophenoxyacetamide, acetoacetamide, (N'-dithiobenzyloxycarbonylamino)acetamide, 3-(p-hydroxyphenyl)propanamide, 3-(o-nitrophenyl)propanamide, 2-methyl-2-(o-nitrophenoxy)propanamide, 2-methyl-2-(o-phenylazophenoxy)propanamide, 4-chlorobutanamide, 3-methyl-3-nitrobutanamide, o-nitrocinnamide, N-acetylmethionine derivative, o-nitrobenzamide, o-(benzoyloxymethyl)benzamide, 4,5-diphenyl-3-oxazolin-2-one, N-phthalimide, N-dithiasuccinimide (Dts), N-2,3-diphenylmaleimide, N-2,5-dimethylpyrrole, N-1,1,4,4-tetramethyldisilylazacyclopentane adduct (STABASE), 5-substituted 1,3-dimethyl-1,3,5-triazacyclohexan-2-one, 5-substituted 1,3-dibenzyl-1,3,5-triazacyclohexan-2-one, 1-substituted 3,5-dinitro-4-pyridone, N-methylamine, N-allylamine, N-[2-trimethylsilyl)ethoxy]methylamine (SEM), N-3-acetoxypropylamine, N-(1-isopropyl-4-nitro-2-oxo-3-pyroolin-3-yl)amine, quaternary ammonium salts, N-benzylamine, N-di(4-methoxyphenyl)methylamine, N-5-dibenzosuberylamine, Ntriphenylmethylamine (Tr), N-[(4-methoxyphenyl)diphenylmethyl]amine (MMTr), N-9-phenylfluorenylamine (PhF), N-2,7-dichloro-9-fluorenylmethyleneamine, N-ferrocenylmethylamino (Fcm), N-2-picolylamino N'-oxide, N-1,1-dimethylthiomethyleneamine, N-benzylideneamine, N-p-methoxybenzylideneamine, N-diphenylmethyleneamine, N-[(2-pyridyl)mesityl]methyleneamine, N-(N',N'-dimethylaminomethylene)amine, N,N'-isopropylidenediamine, N-p-nitrobenzylideneamine, N-salicylideneamine, N-5-chlorosalicylideneamine, N-(5-chloro-2-hydroxyphenyl)phenylmethyleneamine, N-cyclohexylideneamine, N-(5,5-dimethyl-3-oxo-1-cyclohexenyl)amine, N-borane derivative, N-diphenylborinic acid derivative, N-[phenyl(pentacarbonylchromium- or tungsten)carbonyl]amine, N-copper chelate, N-zinc chelate, N-nitroamine, N-nitrosoamine, amine N-oxide, diphenylphosphinamide (Dpp), dimethylthiophosphinamide (Mpt), diphenylthiophosphinamide (Ppt), dialkyl phosphoramidates, dibenzyl phosphoramidate, diphenyl phosphoramidate, benzenesulfenamide, o-nitrobenzenesulfenamide (Nps), 2,4-dinitrobenzenesulfenamide, pentachlorobenzenesulfenamide, 2-nitro-4-methoxybenzenesulfenamide, triphenylmethylsulfenamide, 3-nitropyridinesulfenamide (Npys), p-toluenesulfonamide (Ts), benzenesulfonamide, 2,3,6,-trimethyl-4-methoxybenzenesulfonamide (Mtr), 2,4,6-trimethoxybenzenesulfonamide (Mtb), 2,6-dimethyl-4-methoxybenzenesulfonamide (Pme), 2,3,5,6-tetramethyl-4-methoxybenzenesulfonamide (Mte), 4-methoxybenzenesulfonamide (Mbs), 2,4,6-trimethylbenzenesulfonamide (Mts), 2,6-dimethoxy-4-methylbenzenesulfonamide (iMds), 2,2,5,7,8-pentamethylchroman-6-sulfonamide (Pmc), methanesulfonamide (Ms), β-trimethylsilylethanesulfonamide (SES), 9-anthracenesulfonamide, 4-(4',8'-dimethoxynaphthylmethyl)benzenesulfonamide (DNMBS), benzylsulfonamide, trifluoromethylsulfonamide, and phenacylsulfonamide.

Examples of suitably protected carboxylic acids further include, but are not limited to, silyl-, alkyl-, alkenyl-, aryl-, and arylalkyl-protected carboxylic acids. Examples of suitable silyl groups include trimethylsilyl, triethylsilyl, t-butyldimethylsilyl, t-butyldiphenylsilyl, triisopropylsilyl, and the like. Examples of suitable alkyl groups include methyl, benzyl, p-methoxybenzyl, 3,4-dimethoxybenzyl, trityl, t-butyl, tetrahydropyran-2-yl. Examples of suitable alkenyl groups include allyl. Examples of suitable aryl groups include optionally substituted phenyl, biphenyl, or naphthyl. Examples of suitable arylalkyl groups include optionally substituted benzyl (e.g., p-methoxybenzyl (MPM), 3,4-dimethoxybenzyl, O-nitrobenzyl, p-nitrobenzyl, p-halobenzyl, 2,6-dichlorobenzyl, p-cyanobenzyl), and 2- and 4-picolyl.

In a third aspect the present invention provides a fusion protein comprising the peptide as defined in the first or second aspect of the invention.

In the present invention the term "fusion protein" refers to a protein joining the peptide of the invention to one or more peptide(s) that originally belong to other, separate protein(s).

In one embodiment of the first, second or third aspect of the invention, optionally in combination with any of the embodiments provided above or below, the peptide or fusion protein is conjugated to a moiety, such as a label, a drug, a cell penetration peptide or polyethylene glycol (PEG). There are well-known routine synthetic protocols for conjugating the moiety to the peptide or fusion protein of the invention.

In another embodiment of the first, second or third aspect of the invention, optionally in combination with any of the embodiments provided above or below, the peptide or fusion protein is conjugated to a label. In another embodiment of the first, second or third aspect of the invention, optionally in combination with any of the embodiments provided above or below, the label is conjugated to the N- or C-terminal of the peptide or fusion protein.

A "label" as used herein is a molecule or compound that can be detected by a variety of methods including fluorescence, electrical conductivity, radioactivity, size, and the like. The label may be intrinsically capable of emitting a signal, such as for example fluorescent label that emits light of a particular wavelength following excitation by light of another lower, characteristic wavelength. Alternatively, the label may not be capable of intrinsically emitting a signal but it may be capable of being bound by another compound that does emit a signal. An example of this latter situation is a label such as biotin which itself does not emit a signal but which when bound to labelled avidin or streptavidin molecules can be detected. Other examples of this latter kind of label are ligands that bind specifically to particular receptors. Detectably labelled receptors are allowed to bind to ligand labelled unit specific markers in order to visualize such markers.

Labels that may be used according to the invention include but are not limited to electron spin resonance molecule, a fluorescent molecule, a chemiluminescent molecule, a radioisotope, an enzyme substrate, an enzyme, a biotin molecule, an avidin molecule, an electrical charge transferring molecule, a semiconductor nanocrystal, a semiconductor nanoparticle, a colloid gold nanocrystal, a ligand, a microbead, a magnetic bead, a paramagnetic molecule, a quantum dot, a chromogenic substrate, an affinity molecule, a protein, a peptide, nucleic acid, a carbohydrate, a hapten, an antigen, an antibody, an antibody fragment, and a lipid.

Radioisotopes can be detected with film or charge coupled devices (CCDs), ligands can be detected by binding of a receptor having a fluorescent, chemiluminescent or enzyme tag, and microbeads can be detected using electron or atomic force microscopy.

The conjugation of the label to the peptide can be performed following routine protocols well-known for the skilled in the art.

In another embodiment, the peptide of the first or second aspect of the invention or the fusion protein of the third aspect of the invention, optionally in combination with any of the embodiments provided above or below, is conjugated to a drug. In one embodiment, the drug is conjugated to the N-terminal end of the peptide or protein.

In another embodiment, the peptide of the first or second aspect of the invention or the fusion protein of the third aspect of the invention, optionally in combination with any of the embodiments provided above or below, is conjugated to PEG. In one embodiment, PEG is conjugated to the N-terminal end of the peptide or protein.

The term "PEG" is used here synonymously for polyethylene glycol and derivatives such as derivatives having one methoxylated end, such as methoxy-polyethylene glycol/polyethylene glycol monomethylether. It is known to the skilled artisan that these compounds may both be used for the modification of drugs and differ in the end-group at one end of the PEG chain where the hydroxyl group may be modified with a methoxy group. Furthermore, it is known to a person of skill in the art that the other end of the PEG needs to be modified with specific "activation groups" in order to couple the PEG specifically to a peptide. An overview about such activation groups is given in Zalipsky, Bioconjugate Chem. 1995, 6, 150-165. The molecular weight of PEG used for conjugation can be changed as appropriate for the degree of sustained efficacy required by the resulting PEG-conjugated peptide. The molecular weight of one PEG molecule is from 5 to 40 kDa, such as from 10 to 30 kDa or from 20 to 30 kDa. PEG molecules used for conjugation can have the linear, branched or star-shaped form.

In another embodiment of the first or second aspect of the invention, optionally in combination with any of the embodiments provided above or below, the peptide is conjugated to a cell penetrating peptide.

In present invention the term "cell penetrating peptide" ("CPP") refers to short peptides that facilitate cellular uptake of various molecular cargo (from nanosize particles to small chemical molecules and large fragments of DNA). The "cargo" is associated to peptides via the C(t) or N(t)-end, either through chemical linkage via covalent bonds or through non-covalent interactions. The function of the CPPs are to deliver the cargo into cells, a process that commonly occurs through endocytosis. Current use is limited by a lack of cell specificity in CPP-mediated cargo delivery and insufficient understanding of the modes of their uptake. CPPs typically have an amino acid composition that either contains a high relative abundance of positively charged amino acids such as lysine or arginine or has sequences that contain an alternating pattern of polar/charged amino acids and non-polar, hydrophobic amino acids. These two types of structures are referred to as polycationic or amphipathic, respectively. A third class of CPPs are the hydrophobic peptides, containing only apolar residues, with low net charge or have hydrophobic amino acid groups that are crucial for cellular uptake. The conjugation of the CPP to the peptide provided in the present invention can be performed following well-known routine protocols, such as solid phase synthesis or solution selective capping. (cf. Copolovici D. M. et al., "Cell-Penetrating Peptides: Design, Synthesis, and Applications", 2014, ACS Nano, 2014, 8 (3), pp 1972-1994). In another embodiment of the first aspect of the invention, optionally in combination with any of the embodiments provided above or below, the cell penetrating peptide is a polycationic CPP, polyArg or, alternatively, penetratine.

In a fourth aspect the present invention provides a pharmaceutical composition.

The expression "therapeutically effective amount" as used herein, refers to the amount of a compound (i.e. peptide or pharmaceutically acceptable salt thereof) that, when administered passes to blood stream and is sufficient to prevent development of, or alleviate to some extent, one or more of the symptoms of the disease which is addressed. The particular dose of the peptide administered according to this invention will of course be determined by the particular circumstances surrounding the case, including the compound administered, the route of administration, the particular condition being treated, and the similar considerations.

The term "pharmaceutically acceptable" refers to that excipients or carriers suitable for use in the pharmaceutical technology for preparing compositions with medical use.

The expression "excipients and/or carriers" refers to acceptable materials, compositions or vehicles. Each component must be pharmaceutically acceptable in the sense of being compatible with the other ingredients of the composition. It must also be suitable for use in contact with the tissue or organ of humans and non-human animals without excessive toxicity, irritation, allergic response, immunogenicity or other problems or complications commensurate with a reasonable benefit/risk ratio. Examples of suitable acceptable excipients are solvents, dispersion media, diluents, or other liquid vehicles, dispersion or suspension aids, surface active agents, isotonic agents, thickening or emulsifying agents, preservatives, solid binders, lubricants and the like. Except insofar as any conventional excipient medium is incompatible with a substance or its derivatives, such as by producing any undesirable biological effect or otherwise interacting in a deleterious manner with any other component(s) of the pharmaceutical composition, its use is contemplated to be within the scope of this invention.

The formulations of the pharmaceutical compositions described herein may be prepared by any method known or hereafter developed in the art of pharmacology. In general, such preparatory methods include the step of bringing the active ingredient (the peptide) into association with a excipient and/or one or more other accessory ingredients, and then, if necessary and/or desirable, shaping and/or packaging the product into a desired single- or multi-dose unit.

A pharmaceutical composition of the invention may be prepared, packaged, and/or sold in bulk, as a single unit dose, and/or as a plurality of single unit doses. As used herein, a "unit dose" is discrete amount of the pharmaceutical composition comprising a predetermined amount of the active ingredient.

The relative amounts of the active ingredient (i.e., the peptide as defined in any of the previous aspects and embodiments), the acceptable excipients, and/or any additional ingredients in the composition of the invention will vary, depending upon the identity, size, and/or condition of the subject treated and further depending upon the route by which the composition is to be administered.

Acceptable excipients used in the manufacture of these compositions include, but are not limited to, inert diluents, dispersing and/or granulating agents, surface active agents and/or emulsifiers, disintegrating agents, binding agents, preservatives, buffering agents, lubricating agents, and/or oils. Such excipients may optionally be included in the inventive formulations. Excipients such as cocoa butter and suppository waxes, coloring agents, coating agents, sweetening, flavoring, and perfuming agents can be present in the composition, according to the judgment of the formulator.

Exemplary diluents include, but are not limited to, calcium carbonate, sodium carbonate, calcium phosphate, dicalcium phosphate, calcium sulphate, calcium hydrogen phosphate, sodium phosphate lactose, sucrose, cellulose, microcrystalline cellulose, kaolin, mannitol, sorbitol, inositol, sodium chloride, dry starch, cornstarch, powdered sugar, and combinations thereof.

Exemplary granulating and/or dispersing agents include, but are not limited to, potato starch, corn starch, tapioca starch, sodium starch glycolate, clays, alginic acid, guar gum, citrus pulp, agar, bentonite, cellulose and wood products, natural sponge, cation-exchange resins, calcium carbonate, silicates, sodium carbonate, cross-linked polyvinylpyrrolidone) (crospovidone), sodium carboxymethyl starch (sodium starch glycolate), carboxymethyl cellulose, cross-linked sodium carboxymethyl cellulose (croscarmellose), methylcellulose, pregelatinized starch (starch 1500), microcrystalline starch, water insoluble starch, calcium carboxymethyl cellulose, magnesium aluminum silicate (Veegum), sodium lauryl sulphate, quaternary ammonium compounds, and combinations thereof.

Exemplary surface active agents and/or emulsifiers include, but are not limited to, natural emulsifiers (e.g. acacia, agar, alginic acid, sodium alginate, tragacanth, chondrux, cholesterol, xanthan, pectin, gelatin, egg yolk, casein, wool fat, cholesterol, wax, and lecithin), colloidal clays (e.g. bentonite [aluminium silicate] and Veegum [magnesium aluminium silicate]), long chain amino acid derivatives, high molecular weight alcohols (e.g. stearyl alcohol, cetyl alcohol, oleyl alcohol, triacetin monostearate, ethylene glycol distearate, glyceryl monostearate, and propylene glycol monostearate, polyvinyl alcohol), carbomers (e.g., carboxy polymethylene, polyacrylic acid, acrylic acid polymer, and carboxyvinyl polymer), carrageenan, cellulosic derivatives (e.g., carboxymethylcellulose sodium, powdered cellulose, hydroxymethyl cellulose, hydroxypropyl cellulose, hydroxypropyl methylcellulose, methylcellulose), sorbitan fatty acid esters {e.g., polyoxyethylene sorbitan monolaurate [Tween 20], polyoxyethylene sorbitan [Tween 60], polyoxyethylene sorbitan monooleate [Tween 80], sorbitan monopalmitate [Span 40], sorbitan monostearate [Span 60], sorbitan tristearate [Span 65], glyceryl monooleate, sorbitan monooleate [Span 80]), polyoxyethylene esters (e.g., polyoxyethylene monostearate [Myrj 45], polyoxyethylene hydrogenated castor oil, polyethoxylated castor oil, polyoxymethylene stearate, and Solutol), sucrose fatty acid esters, polyethylene glycol fatty acid esters (e.g., Cremophor), polyoxyethylene ethers, (e.g., polyoxyethylene lauryl ether [Brij 30]), poly(vinyl-pyrrolidone), diethylene glycol monolaurate, triethanolamine oleate, sodium oleate, potassium oleate, ethyl oleate, oleic acid, ethyl laurate, sodium lauryl sulphate, Pluronic F 68, Poloxamer 188, cetrimonium bromide, cetylpyridinium chloride, benzalkonium chloride, docusate sodium, etc. and/or combinations thereof.

Exemplary binding agents include, but are not limited to, starch (e.g., cornstarch and starch paste); gelatin; sugars (e.g., sucrose, glucose, dextrose, dextrin, molasses, lactose, lactitol, mannitol); natural and synthetic gums (e.g., acacia, sodium alginate, extract of Irish moss, panwar gum, ghatti gum, mucilage of isapol husks, carboxymethylcellulose, methylcellulose, ethylcellulose, hydroxyethylcellulose, hydroxypropyl cellulose, hydroxypropyl methylcellulose, microcrystalline cellulose, cellulose acetate, polyvinylpyrrolidone), magnesium aluminum silicate (Veegum), and larch arabogalactan); alginates; polyethylene oxide; polyethylene glycol; inorganic calcium salts; silicic acid; polymethacrylates; waxes; water; alcohol; and combinations thereof.

Exemplary preservatives may include antioxidants, chelating agents, antimicrobial preservatives, antifungal preservatives, alcohol preservatives, acidic preservatives, and other preservatives. Exemplary antioxidants include, but are not limited to, alpha tocopherol, ascorbic acid, acorbyl palmitate, butylated hydroxyanisole, butylated hydroxytoluene, monothioglycerol, potassium metabisulphite, propionic acid, propyl gallate, sodium ascorbate, sodium bisulphite, sodium metabisulphite, and sodium sulphite. Exemplary chelating agents include ethylenediaminetetraacetic acid (EDTA), citric acid monohydrate, disodium edetate, dipotassium edetate, edetic acid, fumaric acid, malic acid, phosphoric acid, sodium edetate, tartaric acid, and trisodium edetate. Exemplary antimicrobial preservatives include, but are not limited to, benzalkonium chloride, benzethonium chloride, benzyl alcohol, bronopol, cetrimide, cetylpyridinium chloride, chlorhexidine, chlorobutanol, chlorocresol, chloroxylenol, cresol, ethyl alcohol, glycerine, hexetidine, imidurea, phenol, phenoxyethanol, phenylethyl alcohol, phenylmercuric nitrate, propylene glycol, and thimerosal. Exemplary antifungal preservatives include, but are not limited to, butyl paraben, methyl paraben, ethyl paraben, propyl paraben, benzoic acid, hydroxybenzoic acid, potassium benzoate, potassium sorbate, sodium benzoate, sodium propionate, and sorbic acid. Exemplary alcohol preservatives include, but are not limited to, ethanol, polyethylene glycol, phenol, phenolic compounds, bisphenol, chlorobutanol, hydroxybenzoate, and phenylethyl alcohol. Exemplary acidic preservatives include, but are not limited to, vitamin A, vitamin C, vitamin E, beta-carotene, citric acid, acetic acid, dehydroacetic acid, ascorbic acid, sorbic acid, and phytic acid. Other preservatives include, but are not limited to, tocopherol, tocopherol acetate, deteroxime mesylate, cetrimide, butylated hydroxyanisol (BHA), butylated hydroxytoluened (BHT), ethylenediamine, sodium lauryl sulphate (SLS), sodium lauryl ether sulphate (SLES), sodium bisulphite, sodium metabisulphite, potassium sulphite, potassium metabisulphite, Glydant Plus, Phenonip, methylparaben, Germall 115, Germaben II, Neolone, Kathon, and Euxyl. In certain embodiments, the preservative is an anti-oxidant. In other embodiments, the preservative is a chelating agent.

Exemplary buffering agents include, but are not limited to, citrate buffer solutions, acetate buffer solutions, phosphate buffer solutions, ammonium chloride, calcium carbonate, calcium chloride, calcium citrate, calcium glubionate, calcium gluceptate, calcium gluconate, D-gluconic acid, calcium glycerophosphate, calcium lactate, propanoic acid, calcium levulinate, pentanoic acid, dibasic calcium phosphate, phosphoric acid, tribasic calcium phosphate, calcium hydroxide phosphate, potassium acetate, potassium chloride, potassium gluconate, potassium mixtures, dibasic potassium phosphate, monobasic potassium phosphate, potassium phosphate mixtures, sodium acetate, sodium bicarbonate, sodium chloride, sodium citrate, sodium lactate, dibasic sodium phosphate, monobasic sodium phosphate, sodium phosphate mixtures, tromethamine, magnesium hydroxide, aluminium hydroxide, alginic acid, pyrogen-free water, isotonic saline, Ringer's solution, ethyl alcohol, and combinations thereof.

Exemplary lubricating agents include, but are not limited to, magnesium stearate, calcium stearate, stearic acid, silica, talc, malt, glyceryl behanate, hydrogenated vegetable oils, polyethylene glycol, sodium benzoate, sodium acetate, sodium chloride, leucine, magnesium lauryl sulphate, sodium lauryl sulphate, and combinations thereof.

Exemplary oils include, but are not limited to, almond, apricot kernel, avocado, babassu, bergamot, black current seed, borage, cade, camomile, canola, caraway, carnauba, castor, cinnamon, cocoa butter, coconut, cod liver, coffee, corn, cotton seed, emu, eucalyptus, evening primrose, fish, flaxseed, geraniol, gourd, grape seed, hazel nut, hyssop, isopropyl myristate, jojoba, kukui nut, lavandin, lavender, lemon, litsea cubeba, macademia nut, mallow, mango seed, meadowfoam seed, mink, nutmeg, olive, orange, orange roughy, palm, palm kernel, peach kernel, peanut, poppy seed, pumpkin seed, rapeseed, rice bran, rosemary, safflower, sandalwood, sasquana, savoury, sea buckthorn, sesame, shea butter, silicone, soybean, sunflower, tea tree, thistle, tsubaki, vetiver, walnut, and wheat germ oils. Exemplary oils include, but are not limited to, butyl stearate, caprylic triglyceride, capric triglyceride, cyclomethicone, diethyl sebacate, dimethicone 360, isopropyl myristate, mineral oil, octyldodecanol, oleyl alcohol, silicone oil, and combinations thereof.

Liquid dosage forms for parenteral administration include, but are not limited to, pharmaceutically acceptable liposomes emulsions, microemulsions, solutions, suspensions, syrups and elixirs. In addition to the active ingredients, the liquid dosage forms may comprise inert diluents commonly used in the art such as, for example, water or other solvents, solubilizing agents and emulsifiers such as ethyl alcohol, isopropyl alcohol, ethyl carbonate, ethyl acetate, benzyl alcohol, benzyl benzoate, propylene glycol, 1,3-butylene glycol, dimethylformamide, oils (in particular, cottonseed, groundnut, corn, germ, olive, castor, and sesame oils), glycerol, tetrahydrofurfuryl alcohol, polyethylene glycols and fatty acid esters of sorbitan, and mixtures thereof. In certain embodiments for parenteral administration, the conjugates of the invention are mixed with solubilizing agents such as polyethoxylated castor oil (e.g. CREMOPHOR^{™}), alcohols, oils, modified oils, glycols, polysorbates, cyclodextrins, polymers, and combinations thereof.

Injectable preparations, for example, sterile injectable aqueous or oleaginous suspensions may be formulated according to the known art using suitable dispersing or wetting agents and suspending agents. The sterile injectable preparation may be a sterile injectable solution, suspension or emulsion in a nontoxic parenterally acceptable diluent or solvent, for example, as a solution in 1,3-butanediol. Among the acceptable vehicles and solvents that may be employed are water, Ringer's solution, U.S.P. and isotonic sodium chloride solution. In addition, sterile, fixed oils are conventionally employed as a solvent or suspending medium. For this purpose any bland fixed oil can be employed including synthetic mono- or diglycerides. In addition, fatty acids such as oleic acid are used in the preparation of injectables. Alternatively, the preparation can be in the form of liposomes.

The injectable formulations can be sterilized, for example, by filtration through a bacterial-retaining filter, or by incorporating sterilizing agents in the form of sterile solid compositions which can be dissolved or dispersed in sterile water or other sterile injectable medium prior to use.

In order to prolong the effect of a drug, it is often desirable to slow the absorption of the drug from subcutaneous or intramuscular injection. This may be accomplished by the use of a liquid suspension of crystalline or amorphous material with poor water solubility. The rate of absorption of the drug then depends upon its rate of dissolution which, in turn, may depend upon crystal size and crystalline form. Alternatively, delayed absorption of a parenterally administered drug form is accomplished by dissolving or suspending the drug in an oil vehicle.

The peptides of the invention can be in micro-encapsulated form with one or more excipients as noted above. In one embodiment, the peptides of the invention are formulated in liposomes.

It will be appreciated that peptides and pharmaceutical compositions of the present invention can be employed in combination therapies. The particular combination of therapies (therapeutics or procedures) to employ in a combination regimen will take into account compatibility of the desired therapeutics and/or procedures and the desired therapeutic effect to be achieved. It will be appreciated that the therapies employed may achieve a desired effect for the same purpose, or they may achieve different effects (e.g., control of any adverse effects).

The pharmaceutical composition of the present invention may be administered either alone or in combination with one or more other therapeutic agents. By "in combination with," it is not intended to imply that the agents must be administered at the same time and/or formulated for delivery together, although these methods of delivery are within the scope of the invention. The compositions can be administered concurrently with, prior to, or subsequent to, one or more other desired therapeutics or medical procedures. In general, each agent will be administered at a dose and/or on a time schedule determined for that agent. Additionally, the invention encompasses the delivery of the peptide or pharmaceutical compositions in combination with agents that may improve their bioavailability, reduce and/or modify their metabolism, inhibit their excretion, and/or modify their distribution within the body.

The particular combination of therapies to employ in a combination regimen will take into account compatibility of the desired therapeutics and/or procedures and/or the desired therapeutic effect to be achieved. It will be appreciated that the therapies employed may achieve a desired effect for the same disorder (for example, a peptide of the invention may be administered concurrently with another biologically active agent used to treat the same disorder), and/or they may achieve different effects (e.g., control of any adverse effects). In will further be appreciated that biologically active agents utilized in this combination may be administered together in a single composition or administered separately in different compositions.

The expression "in combination with" also encompasses the possibility of conjugating (by chemical-physical interactions) the peptide of the invention to any of the further agents mentioned above and below, which can be either a therapeutic agent or an agent for improving the profile of the peptide (such as bioavailability), among others.

In a sixth and seventh aspects the present invention provides the peptides of the first and second aspect of the invention, the fusion protein of the third aspect or the pharmaceutical aspect of the invention for use in the prevention or treatment of a disease caused by a dysregulation of VEGF expression and angiogenesis, respectively.

The term "VEGF expression level" refers either to the level of VEGF protein or mRNA. In one embodiment of the invention, the peptides of the invention promote a regulation of VEGF mRNA. In another embodiment of the invention, the peptides of the invention promote the regulation of VEGF protein There are commercial kits for determining the amount of VEGF at the level of protein or mRNA.

In the context of the invention, the dysregulation of the angiogenesis is due to an alteration in VEGF expression levels. Thus, there are diseases wherein the dysregulation in angiogenesis is due to an increase in VEGF expression. In this situation, the peptides of the first aspect of the invention including a biradical "L" can be useful, as they are able to reduce VEGF expression (see data provided below). Thus, in one embodiment of the sixth and seventh aspect of the invention, the peptide is one according to the first aspect and includes a linker biradical "L" as defined in any of the embodiments provided above, and it is used for the treatment or prevention of a disease caused by an increase in VEGF levels. Illustrative non-limitative examples of diseases caused by an increase in VEGF levels are blindness disorders such as diabetic retinopathy, age-related macular degeneration (AMD), glaucoma, psoriasis, chronic obstructive pulmonary disease and cancer, among others (Cameliet P. 2003).

In other situations, the angiogenesis dysregulation is due to a reduction in VEGF expression. Thus, in an alternative embodiment of the sixth and seventh aspects of the invention, the peptide is the lineal peptide provided under the first aspect of the invention or any of those provided in the second aspect of the invention, and are used for the treatment or prevention of a disease caused by a reduction in VEGF levels. The peptides of the invention would increase the low VEGF levels, stimulating VEGF production (data also provided below). Illustrative non-limitative examples of diseases related by a reduction in angiogenesis and VEGF expression level are pulmonary fibrosis, ischemic tissue injury, gastric ulceration, nephropathy, bone loss, motor neuron degeneration, chronic wound, skin vessel fragility, among others (Manish B. *et al.,* 2010).

Throughout the description and claims the word "comprise" and variations of the word, are not intended to exclude other technical features, additives, components, or steps. Furthermore, the word "comprise" encompasses the case of "consisting of". Additional objects, advantages and features of the invention will become apparent to those skilled in the art upon examination of the description or may be learned by practice of the invention. The following examples are provided by way of illustration, and they are not intended to be limiting of the present invention. Furthermore, the present invention covers all possible combinations of particular and preferred embodiments described herein.

### Examples

### 1. MATERIALS AND METHODS

### Synthetic general procedure

Materials were purchased as following: Fmoc-protected α-amino acids (---); Rinkamide MBHA Resin (Tianjin Nankai HECHENG S&T Co., Ltd); HBTU ((2-(1H-benzotriazol-1-yl)-1,1,3,3-tetramethyluronium hexafluorophosphate), GL Biochem); N-methyl morpholine (Sinopharm Chemical Reagent Co., Ltd.); Succinic anhydride (Aladdin); acetonitrile (Xingke Chemical); ninhydrin (Sinopharm Chemical Reagent Co., Ltd.); Piperidine (Vertellus); Dimethylformamide, DMF (Zhejiang jiangshan chemical co., Ltd); trifluoroacetic acid, TFA (Trifluoroacetic acid, Solvay), TIS (Thioanisole, Solvay)

Briefly, the linear polypeptides were synthesized manually using Fmoc based SPPS (solid phase peptides synthesis) on Rink amide MBHA resin as support.

The following protocol was used:
1. The Fmoc protective group was removed with 20% piperidine in DMF.
2. The resin was washed with DMF five times.
3. The subsequent Fmoc-protected amino acid was coupled for 45 min using Fmoc-AA (3 equiv.), HBTU (3 equiv.), and N-methyl morpholine (6 equiv.).
4. The resin was washed with DMF five times. Coupling was checked by ninhydrin test.
5. Repeat from step 1.
6. N-terminal was capped by reacting with succinic anhydride (10 equiv.) and N-methyl morpholine (10 equiv.).

The peptide was cleaved from the resin and deprotected by exposure to solution F (95% TFA, 2,5% water, 2.5% TIS) and lyophilized.

The lyophilized peptides were purified by reverse phase HPLC using a C18 column (see compounds characterization for details). The peptides were identified by LC-MS-ESI. All the mass spectral data for all the compounds are shown below in Table 1.

Materials were purchased as following: Fmoc-protected α-amino acids (include than the olefinic amino acids Fmoc-[(S)-2-(4 pentenyl)alanine]OH, Fmoc-[(R)-2-(4 pentenyl)alanine]OH, Fmoc-[(S)-2-(7 octenyl)alanine]OH, Fmoc-[(R)-2-(4 pentenyl)alanine]OH, 2-(6-chloro-1-H-benzotriazole-1-yl)-1,1,3,3-tetramethylaminium hexafluorophosphate (TBTU), resins, dimethylformamide (DMF), N,N-diisopropylethylamine (DIEA), trifluoroacetic acid (TFA), 1,2-dichloroethane (DCE), Grubbs Ru(IV) catalyst and piperidine were purchased from different suppliers.

Briefly, the linear polypeptides were synthesized with automatic synthesizer using Fmoc solid phase peptide chemistry. Only the coupling with olefinic amino acids was performed manually after removing the resins from the reactor vessel, as disclosed in the previous section.

The ring-closing metathesis reaction was performed in solution with a first generation Grubbs catalyst after cleaving the linear peptide from the resin, as disclosed by Scott J. M. and colleagues (Scott J.M. et al., "Application of Ring-Closing Metathesis to the Synthesis of Rigidified Amino Acids and Peptides", 1996, J. Am. Chem. Soc., 1996, 118 (40), pp 9606-9614).

The deprotected peptide precipitated with methyl-tert-butyl ether at 4° C. and lyophilized.
The lyophilized peptides were purified by reverse phase HPLC using an Agilent ZORBAX 300SB-C18 5um column (see compounds characterization for details). The peptides were identified by LC-MS-ESI. All the mass spectral data for all the compounds are shown below in Table 1.

### HPLC conditions:

SEQ ID NO: 13: The compound was purified by HPLC-RP (C-18 column; Pump A: 100% H₂O with 0,1% TFA; Pump B 80% Acetonitrile with 0,1% TFA) using a linear gradient 29%-39% of B in 20 minutes (R. T. =10,2). Purity grade 95,16% by HPLC;
SEQ ID NO: 14. The compound was purified by HPLC-RP (C-18 column; Pump A: 100% H₂O with 0,1% TFA; Pump B 80% Acetonitrile with 0,1% TFA) using a linear gradient 40%-50% of B in 20 minutes (R. T. = 6,5-7,8). Purity grade >95% by HPLC;
SEQ ID NO: 15. The compound was purified by HPLC-RP (C-18 column; Pump A: 100% H₂O with 0,1% TFA; Pump B 80% Acetonitrile with 0,1% TFA) using a linear gradient 36%-46% of B in 20 minutes (R. T. = 9,2-10,3). Purity grade >95% by HPLC;
SEQ ID NO: 16. The compound was purified by HPLC-RP (C-18 column; Pump A: 100% H₂O with 0,1% TFA; Pump B 80% Acetonitrile with 0,1% TFA) using a linear gradient 50%-60% of B in 20 minutes (R. T. = 10,3-10,9). Purity grade >95% by HPLC;
SEQ ID NO: 17: The compound was purified by HPLC-RP (C-18 column; Pump A: 100% H₂O with 0,1% TFA; Pump B 80% Acetonitrile with 0,1% TFA) using a linear gradient 35%-55% of B in 20 minutes (R. T. = 12,5-13,1). Purity grade >95% by HPLC;
SEQ ID NO: 18. The compound was purified by HPLC-RP (C-18 column; Pump A: 100% H₂O with 0,1% TFA; Pump B 80% Acetonitrile with 0,1% TFA) using a linear gradient 55%-75% of B in 20 minutes (R. T. = 8,5-9,3). Purity grade >95% by HPLC;
SEQ ID NO: 19. The compound was purified by HPLC-RP (C-18 column; Pump A: 100% H₂O with 0,1% TFA; Pump B 80% Acetonitrile with 0,1% TFA) using a linear gradient 36%-46% of B in 20 minutes (R. T. = 9,9-10,4). Purity grade >95% by HPLC;
SEQ ID NO: 20. The compound was purified by HPLC-RP (-18 column; Pump A: 100% H₂O with 0,1% TFA; Pump B 80% Acetonitrile with 0,1% TFA) using a linear gradient 35%-45% of B in 20 minutes (R. T. = 9,8-10,4). Purity grade >95% by HPLC;
SEQ ID NO: 21. The compound was purified by HPLC-RP (-18 column; Pump A: 100% H₂O with 0,1% TFA; Pump B 80% Acetonitrile with 0,1% TFA) using a linear gradient 45%-55% of B in 20 minutes (R. T. = 13,2-14,4). Purity grade 95,7% by HPLC;
SEQ ID NO: 29 The compound was purified by HPLC-RP (C-18 column; Pump A: 100% H₂O with 0,1% TFA; Pump B 80% Acetonitrile with 0,1% TFA) using a linear gradient 38%-48% of B in 20 minutes (R. T. = 8,9-9,3). Purity grade 95,13% by HPLC;
SEQ ID NO: 30. The compound was purified by HPLC-RP (C-18 column; Pump A: 100% H₂O with 0,1% TFA; Pump B 80% Acetonitrile with 0,1% TFA) using a linear gradient 36%-46% of B in 20 minutes (R. T. = 12,4). Purity grade 95,13% by HPLC;
SEQ ID NO: 31. The compound was purified by HPLC-RP (C-18 column; Pump A: 100% H₂O with 0,1% TFA; Pump B 80% Acetonitrile with 0,1% TFA) using a linear gradient 28%-48% of B in 20 minutes (R. T. = 9,9-10,2). Purity grade 95,13% by HPLC;

**TABLE 1: mass characterization**

| SEQ ID NO: | | MW (1H) | Mass (2H) |
|---|---|---|---|
| 13 | calcul. | 2148.7 | 1075.3 |
| | found | | 1075.3 |
| 14 | calcul. | 1910.5 | 956.2 |
| | found | | 956.1 |
| 15 | calcul. | 1840.3 | 921.1 |
| | found | | 921.0 |
| 16 | calcul. | 1772.2 | 887.1 |
| | found | | 887.0 |
| 17 | Calcul. | 1773.81 | 887.9 |
| | found | | 887.7 |
| 18 | calcul. | 1827.3 | 914.7 |
| | found | | 914.6 |
| 19 | calcul. | 2266.9 | 1134.4 |
| | found | | 1134.3 |
| 20 | calcul. | 2196.7 | 1099.3 |
| | found | | 1099.3 |
| 21 | calcul. | 2128.7 | 1065.3 |
| | found | | 1065.2 |
| 29 | calcul. | 2255.3 | 1128.7 |
| | found | | 1128.8 |
| 30 | calcul. | 2186,47 | 1094.2 |
| | found | | 1094.3 |
| 31 | calcul. | 2130,49 | 1066.2 |
| | found | | 1066.2 |

### Cell line, cell culture and hypoxia induction conditions

MDA-MB-231 human breast cancer cell lines, HaCat immortalized human keratinocyte cell line and ARPE-19 human retinal pigment epithelia (RPE) cell line were purchased from American Type Culture Collection (ATCC, Manassas, VA, USA). Human cells were routinely authenticated using genotype profiling according to the ATCC guidelines. ARPE-19 cells were maintained in DMEM/F12 medium with HEPES supplemented with 10% fetal calf serum and antibiotics. MDA-MB-231 and HaCaT were maintained in DMEM medium with glucose 4.5gr/L, glutamine and pyruvate 2mM and supplemented with 10% fetal calf serum and antibiotics.

Hypoxia was effectively induced in assayed cells treated with CoCl₂. A fresh stock solution 0.4 M CoCl₂ was prepared in water and added to the medium to obtain the concentration of 50 µM for MDA-MB-231, 200 pM for HaCaT and 300 µM for ARPE-19 cells. Cells were incubated with respective CoCl₂ concentration for 24 hr to mimic hypoxic conditions.

### VEGF assay

The VEGF-content of the supernatant of culture cells was measured by a VEGF-ELISA (Abcam; ab100662; Cambridge, UK) following the manufacturer's instructions. The range of detection of the ELISA was between 8.23 pg/ml and 6,000 pg/ml. The ELISA detects all isoforms of human VEGF-A. The assay was performed in untreated cells in normoxia and hypoxia conditions to determine and calculate the ratio between the VEGF amount in hypoxia vs. VEGF amount in normoxia at 24hr, which resulted in >1 in each cell line tested. The same procedure was used in the presence of the peptides of the invention.

### Real Time Quantitative RT-PCR

Extraction of RNA from indicated cells was performed using the NZY total RNA isolation kit (Nzytech, Lisboa, Portugal) and cDNA prepared using the NZY First-Strand cDNA Synthesis Kit (Nzytech). Primers were designed using Primer Blast software (NCBI) and obtained from Thermo-Fisher. mRNA expression levels were evaluated by qRT-PCR (QuantStudio 5 Real-Time PCR System, Applied Biosystems, Foster City, CA, USA) using SYBR green master mix (Applied Biosystems) and normalized to β-actin. The following primers were used in this study: *β-Actin* (forward: 5'-AGA AAA TCT GGC ACC ACA CC-3' (SEQ ID NO: 32); reverse: 5'-GGG GTG TTG AAG GTC TCA AA-3' (SEQ ID NO: 33)); *VGFA* (forward: 5'-TAC TGC CAT CCA ATC GAG AC-3' (SEQ ID NO: 34); reverse: 5'-GCA TGG TGA TGT TGG ACT-3' (SEQ ID NO: 35)).

### Statistical Analysis.

RT-PCR experiments were repeated at least 2 times. For VEGF assays, the mean ± S.D of 3 determinations carried out in triplicate were calculated. Statistical significance was determined using Mann-Whitney tests for comparisons of means in a SPPS statistical software for Microsoft Windows, release 6.0 (Professional Statistic, Chicago, IL, USA). Individual comparisons were made using Student's two-tailed, unpaired t-tests. Criterion for significance was P < 0.05 for all comparisons.

### 2. RESULTS

The objective of this study was to determine the regulation of VEGF by the peptides in hypoxia induced conditions in ARPE-19 cell lines. The VEGF protein (using ELISA kit) was calculated in untreated cells in hypoxia vs. normoxia and a threshold of activity was set. Then, cells were treated with a solution of the corresponding peptide of the invention at 20 µM dissolved in cell culture medium alone (normoxia condition) or in the presence of CoCl₂ (hypoxia conditions) and the ratio of VEGF protein amount was calculated. The peptides showing a VEGF_{hyp}/VEGFₙₒᵣₘ ratio higher than the ratio in untreated cells were considered as inducers of VEGF protein, while peptides providing a VEGF_{hyp}/VEGFₙₒᵣₘ ratio lower than the ratio in untreated cells were considered as reducers of VEGF protein, as reported in table 2.

**Table 2. VEGF_{hyp}/VEGFₙₒᵣₘ in ARPE-19 cells untreated and treated with peptides at 20uM**

| VEGF regulation in ARPE-19 cells (20uM) | |
|---|---|
| Compound | Relative VEGF ( Hipoxia/Normoxia) |
| Untreated | 2.02 |
| SEQ ID NO: 13 | 2.59 |
| SEQ ID NO: 29 | 2.60 |
| SEQ ID NO: 30 | 2.70 |
| SEQ ID NO: 31 | 2.64 |
| SEQ ID NO: 14 | 1.41 |
| SEQ ID NO: 16 | 1.01 |
| SEQ ID NO: 17 | 1.02 |
| SEQ ID NO: 19 | 1.39 |
| SEQ ID NO: 20 | 0.99 |
| SEQ ID NO: 21 | 1.36 |

### Results show that the peptides of the invention are able to remarkably modulate VEGF levels

In particular, the lineal peptide of the first aspect of the invention increased the VEGF value under hypoxia conditions. Surprisingly, when the peptide of the first aspect of the invention included a staple, its behaviour against VEGF remarkably changed, giving rise to a dramatically decrease in VEGF, about 50%. Thus, the inventors have found that the peptide comprising the sequence SEQ ID NO: 1 can modulate VEGF and, consequently, the angiogenesis, by including or not the linker biradical "L" in the sequence SEQ ID NO: 1: the absence of the linker provides a peptide that enhances the VEGF, and consequently "enhances" angiogenesis, whereas the presence of the linker provides a peptide that reduces VEGF, and consequently "reduces" angiogenesis.

In the case of the peptides of the second aspect of the invention, it was found that they were capable of increasing the expression of VEGF under hypoxia conditions with respect to the levels obtained under normoxia conditions. Thus, the peptides of the second aspect of the invention can also be useful to recover the levels of VEGF in those situations wherein a reduction in the growth factor has occurred.

To confirm the results, peptide SEQ ID NO: 16 was tested in a human keratocytes cell line as well as in MDA-MB-231 cell line, which are cell lines wherein the induced hypoxia conditions induce an increase in VEGF protein expression when compared to normoxia. Cells were treated with a solution of the peptide of the invention at 10 µM dissolved in cell culture medium alone (normoxia condition) or in the presence of CoCl₂ (hypoxia conditions) and the ratio of VEGF amount was calculated. Results are reported in Table 3.

**Table 3.**

| VEGF regulation in HaCat cells (10 µM) | |
|---|---|
| **peptide** | Relative VEGF ( Hipoxia/Normoxia) |
| **Untreated** | **1.15** |
| **SEQ ID NO: 16** | 0.88 |

| VEGF regulation in MDA-MB-231 cells (10 µM) | |
|---|---|
| **Untreated** | 1.72 |
| **SEQ ID NO: 16** | 1.30 |

Results show that the peptides of the invention reduced VEGF ratio, being consistent with those provided ARPE-19 cells.

The VEGF expression regulation was also determined by RP-PCR in ARPE-19 cells treated with the peptide SEQ ID NO: 17 at 10 µM for 24 hr in normoxia and hypoxia conditions. Results are reported in Table 4.

**Table 4. VEGF mRNA in ARPE-19 cells untreated and treated with peptide at 10 µM**

| | **VEGF mRNA Copy/Copy million of Actine** | **Relative Copy to Control** |
|---|---|---|
| **Untreated Normoxia** | 33918 | 1 |
| **Untreated Hypoxia** | 152782 | 4.50 |
| **SEQ ID NO: 17 Normoxia** | 31454 | 0.93 |
| **SEQ ID NO: 17 Hypoxia** | 25873 | 0.76 |

When cells were in hypoxia conditions a clear increment in the levels of VEGF mRNA was detected. Results show that treatment with the peptide SEQ ID NO: 17 reduced significantly the mRNA level when compared to untreated cells in hypoxia, while just a slide reduction was detected in normoxia.

### Citation List

Altschul et al., "Basic local alignment search tool", 1990, J. Mol. Biol, v. 215, pages 403-410.
Carmeliet P., "Angiogenesis in health and disease", Nature Medicine, 2003, 9, 653-660.
Copolovici D. M. et al., "Cell-Penetrating Peptides: Design, Synthesis, and Applications", 2014, ACS Nano, 2014, 8 (3), pp 1972-1994.
Higgins et al., "CLUSTAL V: improved software for multiple sequence alignment", 1992, CABIOS, 8(2), pages 189-191.
Kim Young-Woo et al., "Synthesis of all-hydrocarbon stapled a-helical peptides by ring-closing olefin metathesis", Nature Protocols, 2011, 6(6), p. 761-771.
Kolb H.C. et al., "The growing impact of click chemistry on drug discovery", 2003, Drug Discov Today, 8(24):1128-1137.
Manisha B. et al., "Angiogenesis: Future of pharmacological modulation", Indian J. Pharmacol., 2010, 42(1), pages 2-8.
Scott J. M. et al., "Application of Ring-Closing Metathesis to the Synthesis of Rigidified Amino Acids and Peptides", J. Am. Chem. Soc., 1996, v.118 (40), pp 9606-9614.
Zalipsky S., "Functionalized Poly(ethylene glycols) for Preparation of Biologically Relevant Conjugates", Bioconjugate Chemistry, 1995, 6 (2), 150-165.

### Clauses

For reasons of completeness, various aspects of the invention are set out in the following numbered clauses:
Clause 1. A peptide or a pharmaceutical salt thereof having a length from 14 to 50 and comprising a sequence having at least a 85% of sequence identity with respect to SEQ ID NO: 1:
   (Leu)m-(Asp)n-Lys-Ala-Ser-Val-Met-Arg-Leu-Thr-Ile-Ser-Tyr-Leu-Arg-Val-(Arg)p-(Lys)q
   wherein
   - "m", "n", "p", and "q" represent integers and are selected from 0 and 1;
   - C-terminal end corresponds to -C(O)R₄; and
   - N-terminal end corresponds to -NHR₅;
   the peptide optionally comprising a linker biradical "L" of formula (I)

   -[(R₁)ₐ-(R₂)-(R₃)_{b}]_{c}- (I)

   which connects an alpha carbon atom of an amino acid located at position "i" with an alpha carbon atom of an amino acid located at position "i+4" or "i+7" in the peptide sequence SEQ ID NO: 1,
   wherein
   "a" and "b" are the same or different and are 0 or 1;
   "c" is comprised from 1 to 10;
   R₁ and R₃ are biradicals independently selected from the group consisting of: (C₁-C₁₀)alkyl; (C₁-C₁₀)alkyl substituted by one or more radicals selected from the group consisting of: halogen, (C₁-C₁₀)alkyl, -OR₆, -NR₇R₈, -SR₉, -SOR₁₀, -SO₂R₁₁, and -CO₂R₁₂; (C₂-C₁₀)alkenyl; (C₂-C₁₀)alkenyl substituted by one or more radicals selected from the group consisting of: halogen, (C₁-C₁₀)alkyl, - OR₆, -NR₇R₈, -SR₉, -SOR₁₀, -SO₂R₁₁, and -CO₂R₁₂; (C₂-C₁₀)alkynyl; and (C₂-C₁₀)alkynyl substituted by one or more radicals selected from the group consisting of: halogen, (C₁-C₁₀)alkyl, -OR₆, - NR₇R₈, -SR₉, -SOR₁₀, -SO₂R₁₁, and -CO₂R₁₂;
   R₂ is a biradical selected from the group consisting of: -O-, C(=O), C(=O)NR₁₃, C(=O)O, S(=O), S(=O)₂, NR₁₄, (C₁-C₁₀)alkyl, (C₂-C₁₀)alkenyl, (C₂-C₁₀)alkynyl, -NR₁₅-NR₁₆-, -N=N-, -S-S-, and a known ring system comprising from 3 to 14 members, the system comprising from 1 to 3 rings, where:
      each one of the rings is saturated, partially unsaturated, or aromatic;
      the rings are isolated, partially or totally fused,
      each one of the members forming the known ring system is selected from the group consisting of: -CH-, -CH₂-, -NH-, -N-, -SH-, -S-, and -O-; and
      the ring system is optionally substituted by one or more radicals independently selected from the group consisting of halogen, -OH, -NO₂, (C₁-C₁₀)alkyl, (C₁-C₁₀)haloalkyl, and (C₁-C₁₀)alkyl-O-; and
   R₄ is a radical selected from the group consisting of -OH and -NR₁₇R₁₈;
   R₅ is a radical selected from the group consisting of -H and (C₁-C₂₀)alkyl;
   R₆, R₇, R₈, R₉, R₁₀, R₁₁, R₁₂, R₁₃, R₁₄, R₁₅, R₁₆, R₁₇ and R₁₈ are radicals independently selected from the group consisting of: -H and (C₁-C₁₀)alkyl; and
   the amino acids which are connected by the linker being of formula (II) 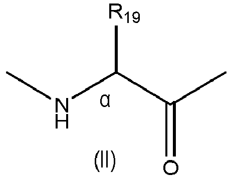 wherein
      R₁₉ is a monoradical selected from the group consisting of: (C₁-C₁₀)alkyl, (C₂-C₁₀)alkenyl, (C₂-C₁₀)alkynyl, and a known ring system comprising from 3 to 14 members, the system comprising from 1 to 3 rings, where:
      each one of the rings is saturated, partially unsaturated, or aromatic;
      the rings are isolated, partially or totally fused,
      each one of the members forming the known ring system is selected from the group consisting of: - CH-, -CH₂-, -NH-, -N-, -SH-, -S-, and -O-.
Clause 2. The peptide according to clause 1, which consists of the sequence SEQ ID NO: 1, optionally including the linker biradical "L" of formula (I) as defined in clause 1.
Clause 3. The peptide according to any one of the previous clauses, which comprises the linker biradical "L" of formula (I) between the alpha carbon atom of the amino acid located at position "i" and the alpha carbon atom of the amino acid located at position "i+7" in the peptide sequence SEQ ID NO: 1.
Clause 4. The peptide according to any one of the previous clauses, which is selected from the group consisting of:
   SEQ ID NO: 2 (Leu)m-(Asp)n-Lys-Ala-**Ser**-Val-Met-Arg-Leu-Thr-Ile-**Ser**-Tyr-Leu-Arg-Val-(Arg)p-(Lys)q;
   SEQ ID NO: 3 (Leu)m-(Asp)n-Lys-Ala-Ser-Val-**Met**-Arg-Leu-Thr-Ile-Ser-Tyr-**Leu**-Arg-Val-(Arg)p-(Lys)q;
   SEQ ID NO: 4: (Leu)m-(Asp)n-Lys-Ala-Ser-Val-Met-**Arg**-Leu-Thr-Ile-Ser-Tyr-Leu-**Arg**-Val-(Arg)p-(Lys)q;
   SEQ ID NO: 5 (Leu)m-(Asp)n-Lys-Ala-Ser-Val-Met-Arg-Leu-**Thr**-Ile-Ser-Tyr-Leu-Arg-Val-**Arg**-(Lys)q;
   SEQ ID NO: 6: Lys-Ala-**Ser**-Val-Met-Arg-Leu-Thr-Ile-**Ser**-Tyr-Leu-Arg-Val-Arg;
   SEQ ID NO: 7: Lys-Ala-Ser-Val-**Met**-Arg-Leu-Thr-Ile-Ser-Tyr-**Leu**-Arg-Val-Arg;
   SEQ ID NO: 8: Lys-Ala-Ser-Val-Met-**Arg**-Leu-Thr-Ile-Ser-Tyr-Leu-**Arg**-Val-Arg;
   SEQ ID NO: 9: Lys-Ala-Ser-Val-Met-Arg-Leu-**Thr**-Ile-Ser-Tyr-Leu-Arg-Val-**Arg**;
   SEQ ID NO: 10: Leu-Asp-Lys-Ala-**Ser**-Val-Met-Arg-Leu-Thr-Ile-**Ser**-Tyr-Leu-Arg-Val-Arg-Lys;
   SEQ ID NO: 11: Leu-Asp-Lys-Ala-Ser-Val-**Met**-Arg-Leu-Thr-Ile-Ser-Tyr-**Leu**-Arg-Val-Arg-Lys; and
   SEQ ID NO: 12: Leu-Asp-Lys-Ala-Ser-Val-Met-**Arg**-Leu-Thr-Ile-Ser-Tyr-Leu-**Arg**-Val-Arg-Lys;
   wherein amino acids marked in bold and underlined, having the same (being both L-amino acids or D-amino acids) or different configuration, point out the location of the linker biradical "L", C-terminal end corresponds to -C(O)R₄; and N-terminal end corresponds to -NHR₅.
Clause 5. The peptide according to any one of the previous clauses, wherein "a", "b", and "c" are 1.
Clause 6. The peptide according to any one of the previous clauses, wherein R₁ and R₃ are biradicals independently selected from the group consisting of: (C₁-C₁₀)alkyl; (C₂-C₁₀)alkenyl; and (C₂-C₁₀)alkynyl.
Clause 7. The peptide according to any one of the previous clauses, wherein R₂ is a biradical selected from the group consisting of: (C₁-C₁₀)alkyl, (C₂-C₁₀)alkenyl, and (C₂-C₁₀)alkynyl.
Clause 8. The peptide according to any one of the previous clauses, wherein R₁₉ is a monoradical selected from the group consisting of: (C₁-C₁₀)alkyl, (C₂-C₁₀)alkenyl, and (C₂-C₁₀)alkynyl.
Clause 9. The peptide according to any one of the previous clauses, wherein:
   - R₁, R₃ and R₁₉ are (C₁-C₁₀)alkyl; R₂ is (C₂-C₁₀)alkenyl; "m" and "n" are the same; and "p" and "q" are the same; or, alternatively,
   - R₁, R₃ and R₁₉ are (C₁-C₁₀)alkyl; R₂ is (C₂-C₁₀)alkenyl; "m" and "n" are the same; and "p" and "q" are different; or, alternatively,
   - R₁, R₃ and R₁₉ are (C₁-C₁₀)alkyl; R₂ is (C₂-C₁₀)alkenyl; "m", "n", "p" and "q" are the same, particularly 1; or, alternatively,
   - R₁, R₃ and R₁₉ are (C₁-C₁₀)alkyl; R₂ is (C₂-C₁₀)alkenyl; "m", "n", "q" are the same and "p" is different; particularly m=n=q=0, and p= 1.
Clause 10. The peptide according to any one of the previous clauses, wherein the C-terminal end corresponds to -C(O)OH or -C(O)NH_{2,} and the N-terminal end corresponds to -NH₂.
Clause 11. The peptide according to any one of the previous clauses, which has a sequence identity of at least 85, 86, 87, 88, 89, 90, 91, 92, 93, 94, 95, 96, 97, 98, 99 or of 100% with respect to a peptide selected from the group consisting of sequences SEQ ID NO: 13 to 21.
Clause12. The peptide according to any one of the previous clauses, which is conjugated to a label, drug, a cell penetrating peptide or polyethylene glycol (PEG).
Clause 13. A fusion protein comprising the peptide as defined in any one of the previous clauses.
Clause 14. A pharmaceutical composition comprising a therapeutically effective amount of the peptide or a pharmaceutical salt thereof as defined in any one of the clauses 1-12, or the fusion protein as defined in clause 13, together with acceptable pharmaceutical excipients and/or carriers.
Clause 15. A peptide or a pharmaceutical salt thereof as defined in any one of the clauses 1-12 or a fusion protein as defined in clause 13 or a pharmaceutical composition as defined in clause 14 for use in therapy.
Clause 16. A peptide or a pharmaceutical salt thereof as defined in any one of the clauses 1-12 or a fusion protein as defined in clause 13 or the pharmaceutical composition as defined in clause 14 for use in the treatment or prevention of a disease caused by a dysregulation of the expression of VEGF, particularly caused by an increase or reduction in the expression of VEGF.
Clause 17. A peptide or a pharmaceutical salt thereof as defined in any one of the clauses 1-12 or a fusion protein as defined in clause 13 or the pharmaceutical composition as defined in clause 14 for use in the treatment or prevention of a disease associated to a dysregulation of the angiogenesis based on the expression of VEGF.
Clause 18. A peptide or a pharmaceutical salt thereof having a length from 15 to 50 and comprising a sequence having at least a 85% of sequence identity with respect to SEQ ID NO: 22:
   (Arg)r-(Ser)s-Arg-Arg-Ser-Lys-Glu-Ser-Glu-Val-Phe-Tyr-Glu-Leu-Ala-His-Gln-(Leu)t-(Pro)v
   wherein
   - "r", "s", "t", and "v" represent integers and are selected from 0 and 1;
   - C-terminal end corresponds to -C(O)R₄;
   - N-terminal end corresponds to -NHR₅;
   the peptide optionally comprising a linker biradical "L" of formula (I)

   -[(R₁)ₐ-(R₂)-(R₃)_{b}]_{c}- (I)

   which is connecting an alpha carbon atom of an amino acid located at position "I" with an alpha carbon atom of an amino acid located at position "i+4" or "i+7" in the peptide sequence SEQ ID NO: 21,
   wherein
   "a" and "b" are the same or different and are 0 or 1;
      "c" is comprised from 1 to 10;
   R₁ and R₃ are biradicals independently selected from the group consisting of: (C₁-C₁₀)alkyl; (C₁-C₁₀)alkyl substituted by one or more radicals selected from the group consisting of: halogen, (C₁-C₁₀)alkyl, -OR₆, -NR₇R₈, -SR₉, -SOR₁₀, -SO₂R₁₁, and -CO₂R₁₂; (C₂-C₁₀)alkenyl; (C₂-C₁₀)alkenyl substituted by one or more radicals selected from the group consisting of: halogen, (C₁-C₁₀)alkyl, - OR₆, -NR₇R₈, -SR₉, -SOR₁₀, -SO₂R₁₁, and -CO₂R₁₂; (C₂-C₁₀)alkynyl; and (C₂-C₁₀)alkynyl substituted by one or more radicals selected from the group consisting of: halogen, (C₁-C₁₀)alkyl, -OR₆, - NR₇R₈, -SR₉, -SOR₁₀, -SO₂R₁₁, and -CO₂R₁₂;
   R₂ is a biradical selected from the group consisting of: -O-, C(=O), C(=O)NR₁₃, C(=O)O, S(=O), S(=O)₂, NR₁₄, (C₁-C₁₀)alkyl, (C₂-C₁₀)alkenyl, (C₂-C₁₀)alkynyl, -NR₁₅-NR₁₆-, -N=N-, -S-S-, and a known ring system comprising from 3 to 14 members, the system comprising from 1 to 3 rings, where:
      each one of the rings is saturated, partially unsaturated, or aromatic;
      the rings are isolated, partially or totally fused,
      each one of the members forming the known ring system is selected from the group consisting
      of: -CH-, -CH₂-, -NH-, -N-, -SH-, -S-, and -O-; and
      the ring system is optionally substituted by one or more radicals independently selected from the group consisting of halogen, -OH, -NO₂, (C₁-C₁₀)alkyl, (C₁-C₁₀)haloalkyl, and (C₁-C₁₀)alkyl-O-; and
   R₄ is a radical selected from the group consisting of -OH and -NR₁₇R₁₈;
      R₅ is a radical selected from the group consisting of -H and (C₁-C₂₀)alkyl;
      R₆, R₇, R₆, R₉, R₁₀, R₁₁, R₁₂, R₁₃, R₁₄, R₁₅, R₁₆, R₁₇ and R₁₈ are radicals independently selected from the group consisting of: -H and (C₁-C₁₀)alkyl; and
   the amino acids which are connected by the linker being of formula (II) 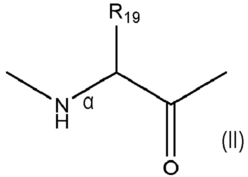 wherein
      R₁₉ is a monoradical selected from the group consisting of: (C₁-C₁₀)alkyl, (C₂-C₁₀)alkenyl, (C₂-C₁₀)alkynyl, and a known ring system comprising from 3 to 14 members, the system comprising from 1 to 3 rings, where:
      each one of the rings is saturated, partially unsaturated, or aromatic;
      the rings are isolated, partially or totally fused,
      each one of the members forming the known ring system is selected from the group consisting of: - CH-, -CH₂-, -NH-, -N-, -SH-, -S-, and -O-.
Clause 19. The peptide according to clause 18, which consists of the sequence SEQ ID NO: 21, optionally including the linker biradical "L" of formula (I) as defined in clause 18.
Clause 20. The peptide according to any one of the previous clauses 18-19, which comprises the linker biradical "L" of formula (I) between the alpha carbon atom of the amino acid located at position "i" and the alpha carbon atom of the amino acid located at position "i+7" in the peptide sequence SEQ ID NO: 21.
Clause 21. The peptide according to any one of the previous clauses 18-20, which is selected from the group consisting of:
   SEQ ID NO: 23 (Arg)r-(Ser)s-Arg-Arg-Ser-Lys-Glu-**Ser**-Glu-Val-Phe-Tyr-Glu-Leu-**Ala**-His-Gln-(Leu)t-(Pro)v;
   SEQ ID NO: 24 (Arg)r-(Ser)s-Arg-Arg-Ser-Lys-Glu-Ser-Glu-**Val**-Phe-Tyr-Glu-Leu-Ala-His-**Gln**-(Leu)-(Pro)v;
   SEQ ID NO: 25: (Arg)r-(Ser)s-Arg-Arg-**Ser**-Lys-Glu-Ser-Glu-Val-Phe-**Tyr**-Glu-Leu-Ala-His-Gln-(Leu)t-(Pro)v;
   SEQ ID NO: 26: Arg-Ser-Arg-Arg-Ser-Lys-Glu-**Ser**-Glu-Val-Phe-Tyr-Glu-Leu-**Ala**-His-Gln;
   SEQ ID NO: 27: Arg-Ser-Arg-Arg-Ser-Lys-Glu-Ser-Glu-**Val**-Phe-Tyr-Glu-Leu-Ala-His-**Gln**; and
   SEQ ID NO: 28: Arg-Arg-**Ser**-Lys-Glu-Ser-Glu-Val-Phe-**Tyr**-Glu-Leu-Ala-His-Gln-Leu-Pro,
   wherein amino acids marked in bold and underlined, having the same (being both L-amino acids or D-amino acids) or different configuration, point out the location of the linker biradical "L", C-terminal end corresponds to -C(O)R₄; and N-terminal end corresponds to -NHR₅.
Clause 22. The peptide according to any one of the previous clauses 18-21, wherein "a", "b", and "c" are 1.
Clause 23. The peptide according to any one of the previous clauses 18-22, wherein R₁ and R₃ are biradicals independently selected from the group consisting of: (C₁-C₁₀)alkyl; (C₂-C₁₀)alkenyl; and (C₂-C₁₀)alkynyl.
Clause 24 The peptide according to any one of the previous clauses 18-23, wherein R₂ is a biradical selected from the group consisting of: (C₁-C₁₀)alkyl, (C₂-C₁₀)alkenyl, and (C₂-C₁₀)alkynyl.
Clause 25. The peptide according to any one of the previous clauses 18-24, wherein R₁₉ is a monoradical selected from the group consisting of: (C₁-C₁₀)alkyl, (C₂-C₁₀)alkenyl, and (C₂-C₁₀)alkynyl.
Clause 26. The peptide according to any one of the previous clauses 18-25, wherein
   - R₁, R₃ and R₁₉ are (C₁-C₁₀)alkyl; R₂ is (C₂-C₁₀)alkenyl; "r" and "s" are the same; and "t" and "v" are the same; or alternatively,
   - R₁, R₃ and R₁₉ are (C₁-C₁₀)alkyl; R₂ is (C₂-C₁₀)alkenyl; "r" and "s" are the same and represent 1; and "t" and "v" are the same and represent 0; or alternatively
   - R₁, R₃ and R₁₉ are (C₁-C₁₀)alkyl; R₂ is (C₂-C₁₀)alkenyl; "r" and "s" are the same and represent 0; and "t" and "v" are the same and represent 1.
Clause 27. The peptide according to any one of the previous clauses 18-26, wherein the C-terminal end corresponds to -C(O)OH or -C(O)NH_{2,} particularly -C(O)NH_{2,} and the N-terminal end corresponds to -NH₂.
Clause 28. The peptide of according to any one of the previous clauses 18-27, which has a sequence identity of at least 85, 86, 87, 88, 89, 90, 91, 92, 93, 94, 95, 96, 97, 98, 99 or of 100% with respect to a peptide selected from the group consisting of sequences SEQ ID NO: 29 to 31.
Clause 29. The peptide according to any one of the previous clauses 18-28, which is conjugated to a label, drug, cell penetrating peptide or PEG.
Clause 30. A fusion protein comprising the peptide as defined in any one of the previous clauses 18-29.
Clause 31. A pharmaceutical composition comprising a therapeutically effective amount of the peptide or a pharmaceutical salt thereof as defined in any one of the clauses 18-29, or the fusion protein as defined in clause 30, together with acceptable pharmaceutical excipients and/or carriers.
Clause 32. A peptide or a pharmaceutical salt thereof as defined in any one of the clauses 18-29 or a fusion protein as defined in clause 30 or a pharmaceutical composition as defined in clause 31 for use in therapy.
Clause 33. A peptide or a pharmaceutical salt thereof as defined in any one of the claims 18-29 or a fusion protein as defined in claim 30 or the pharmaceutical composition as defined in claim 31 for use in the treatment or prevention of a disease cause by a dysregulation of the expression of VEGF.
Clause 34. A peptide or a pharmaceutical salt thereof as defined in any one of the clauses 18-29 or a fusion protein as defined in clause 30 or the pharmaceutical composition as defined in clause 31 for use in in the treatment or prevention of a disease associated to a dysregulation of the angiogenesis based on the expression level of VEGF.

## Claims

1. A peptide or a pharmaceutical salt thereof having a length from 14 to 50 and comprising a sequence having at least a 85% of sequence identity with respect to SEQ ID NO: 1:
(Leu)m-(Asp)n-Lys-Ala-Ser-Val-Met-Arg-Leu-Thr-Ile-Ser-Tyr-Leu-Arg-Val-(Arg)p-(Lys)q
wherein
- "m", "n", "p", and "q" represent integers and are selected from 0 and 1;
- C-terminal end corresponds to -C(O)R₄; and
- N-terminal end corresponds to -NHR₅;
the peptide optionally comprising a linker biradical "L" of formula (I)
-[(R₁)ₐ-(R₂)-(R₃)_{b}]_{c}- (I)
which connects an alpha carbon atom of an amino acid located at position "i" with an alpha carbon atom of an amino acid located at position "i+4" or "i+7" in the peptide sequence SEQ ID NO: 1,
wherein
"a" and "b" are the same or different and are 0 or 1;
"c" is comprised from 1 to 10;
R₁ and R₃ are biradicals independently selected from the group consisting of: (C₁-C₁₀)alkyl; (C₁-C₁₀)alkyl substituted by one or more radicals selected from the group consisting of: halogen, (C₁-C₁₀)alkyl, -OR₆, -NR₇R₈, -SR₉, -SOR₁₀, -SO₂R₁₁, and -CO₂R₁₂; (C₂-C₁₀)alkenyl; (C₂-C₁₀)alkenyl substituted by one or more radicals selected from the group consisting of: halogen, (C₁-C₁₀)alkyl, - OR₆, -NR₇R₈, -SR₉, -SOR₁₀, -SO₂R₁₁, and -CO₂R₁₂; (C₂-C₁₀)alkynyl; and (C₂-C₁₀)alkynyl substituted by one or more radicals selected from the group consisting of: halogen, (C₁-C₁₀)alkyl, -OR₆, - NR₇R₈, -SR₉, -SOR₁₀, -SO₂R₁₁, and -CO₂R₁₂;
R₂ is a biradical selected from the group consisting of: -O-, C(=O), C(=O)NR₁₃, C(=O)O, S(=O), S(=O)₂, NR₁₄, (C₁-C₁₀)alkyl, (C₂-C₁₀)alkenyl, (C₂-C₁₀)alkynyl, -NR₁₅-NR₁₆-, -N=N-, -S-S-, and a known ring system comprising from 3 to 14 members, the system comprising from 1 to 3 rings, where:
each one of the rings is saturated, partially unsaturated, or aromatic;
the rings are isolated, partially or totally fused,
each one of the members forming the known ring system is selected from the group consisting of: -CH-, -CH₂-, -NH-, -N-, -SH-, -S-, and -O-; and
the ring system is optionally substituted by one or more radicals independently selected from the group consisting of halogen, -OH, -NO₂, (C₁-C₁₀)alkyl, (C₁-C₁₀)haloalkyl, and (C₁-C₁₀)alkyl-O-; and
R₄ is a radical selected from the group consisting of -OH and -NR₁₇R₁₈;
R₅ is a radical selected from the group consisting of -H and (C₁-C₂₀)alkyl;
R₆, R₇, R₈, R₉, R₁₀, R₁₁, R₁₂, R₁₃, R₁₄, R₁₅, R₁₆, R₁₇ and R₁₈ are radicals independently selected from the group consisting of: -H and (C₁-C₁₀)alkyl; and
the amino acids which are connected by the linker being of formula (II) 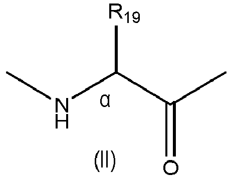 wherein
R₁₉ is a monoradical selected from the group consisting of: (C₁-C₁₀)alkyl, (C₂-C₁₀)alkenyl, (C₂-C₁₀)alkynyl, and a known ring system comprising from 3 to 14 members, the system comprising from 1 to 3 rings, where:
each one of the rings is saturated, partially unsaturated, or aromatic;
the rings are isolated, partially or totally fused,
each one of the members forming the known ring system is selected from the group consisting of: - CH-, -CH₂-, -NH-, -N-, -SH-, -S-, and -O-.

2. The peptide according to claim 1, which consists of the sequence SEQ ID NO: 1, optionally including the linker biradical "L" of formula (I) as defined in claim 1.

3. The peptide according to any one of the previous claims, which comprises the linker biradical "L" of formula (I) between the alpha carbon atom of the amino acid located at position "i" and the alpha carbon atom of the amino acid located at position "i+7" in the peptide sequence SEQ ID NO: 1.

4. The peptide according to any one of the previous claims, which is selected from the group consisting of:
SEQ ID NO: 2 (Leu)m-(Asp)n-Lys-Ala-**Ser**-Val-Met-Arg-Leu-Thr-Ile-**Ser**-Tyr-Leu-Arg-Val-(Arg)p-(Lys)q;
SEQ ID NO: 3 (Leu)m-(Asp)n-Lys-Ala-Ser-Val-**Met**-Arg-Leu-Thr-Ile-Ser-Tyr-**Leu**-Arg-Val-(Arg)p-(Lys)q;
SEQ ID NO: 4: (Leu)m-(Asp)n-Lys-Ala-Ser-Val-Met-**Arg**-Leu-Thr-Ile-Ser-Tyr-Leu-**Arg**-Val-(Arg)p-(Lys)q;
SEQ ID NO: 5 (Leu)m-(Asp)n-Lys-Ala-Ser-Val-Met-Arg-Leu-**Thr**-Ile-Ser-Tyr-Leu-Arg-Val-**Arg**-(Lys)q;
SEQ ID NO: 6: Lys-Ala-**Ser**-Val-Met-Arg-Leu-Thr-Ile-**Ser**-Tyr-Leu-Arg-Val-Arg;
SEQ ID NO: 7: Lys-Ala-Ser-Val-**Met**-Arg-Leu-Thr-Ile-Ser-Tyr-**Leu**-Arg-Val-Arg;
SEQ ID NO: 8: Lys-Ala-Ser-Val-Met-**Arg**-Leu-Thr-Ile-Ser-Tyr-Leu-**Arg**-Val-Arg;
SEQ ID NO: 9: Lys-Ala-Ser-Val-Met-Arg-Leu-**Thr**-Ile-Ser-Tyr-Leu-Arg-Val-**Arg**;
SEQ ID NO: 10: Leu-Asp-Lys-Ala-**Ser**-Val-Met-Arg-Leu-Thr-Ile-**Ser**-Tyr-Leu-Arg-Val-Arg-Lys;
SEQ ID NO: 11: Leu-Asp-Lys-Ala-Ser-Val-**Met**-Arg-Leu-Thr-Ile-Ser-Tyr-**Leu**-Arg-Val-Arg-Lys; and
SEQ ID NO: 12: Leu-Asp-Lys-Ala-Ser-Val-Met-**Arg**-Leu-Thr-Ile-Ser-Tyr-Leu-**Arg**-Val-Arg-Lys;
wherein amino acids marked in bold and underlined point out the location of the linker biradical "L", C-terminal end corresponds to -C(O)R₄; and N-terminal end corresponds to -NHR₅.

5. The peptide according to any one of the previous claims, wherein "a", "b", and "c" are 1.

6. The peptide according to any one of the previous claims, wherein R₁ and R₃ are biradicals independently selected from the group consisting of: (C₁-C₁₀)alkyl; (C₂-C₁₀)alkenyl; and (C₂-C₁₀)alkynyl.

7. The peptide according to any one of the previous claims, wherein R₂ is a biradical selected from the group consisting of: (C₁-C₁₀)alkyl, (C₂-C₁₀)alkenyl, and (C₂-C₁₀)alkynyl.

8. The peptide according to any one of the previous claims, wherein R₁₉ is a monoradical selected from the group consisting of: (C₁-C₁₀)alkyl, (C₂-C₁₀)alkenyl, and (C₂-C₁₀)alkynyl.

9. The peptide according to any one of the previous claims, wherein:
- R₁, R₃ and R₁₉ are (C₁-C₁₀)alkyl; R₂ is (C₂-C₁₀)alkenyl; "m" and "n" are the same; and "p" and "q" are the same; or, alternatively,
- R₁, R₃ and R₁₉ are (C₁-C₁₀)alkyl; R₂ is (C₂-C₁₀)alkenyl; "m" and "n" are the same; and "p" and "q" are different; or, alternatively,
- R₁, R₃ and R₁₉ are (C₁-C₁₀)alkyl; R₂ is (C₂-C₁₀)alkenyl; "m", "n", "p" and "q" are the same; or, alternatively,
- R₁, R₃ and R₁₉ are (C₁-C₁₀)alkyl; R₂ is (C₂-C₁₀)alkenyl; "m", "n", "q" are the same and "q" is different.

10. The peptide according to any one of the previous claims, wherein the C-terminal end corresponds to -C(O)OH or -C(O)NH₂ and the N-terminal end corresponds to -NH₂.

11. The peptide of according to any one of the previous claims, which has a sequence identity of at least 85, 86, 87, 88, 89, 90, 91, 92, 93, 94, 95, 96, 97, 98, 99 or of 100% with respect to a peptide selected from the group consisting of sequences SEQ ID NO: 13 to 21.

12. The peptide according to any one of the previous claims, which is conjugated to a label, drug, a cell penetrating peptide or polyethylene glycol.

13. A fusion protein comprising the peptide as defined in any one of the previous claims.

14. A pharmaceutical composition comprising a therapeutically effective amount of the peptide or a pharmaceutical salt thereof as defined in any one of the claims 1-12, or the fusion protein as defined in claim 13, together with acceptable pharmaceutical excipients and/or carriers.

15. A peptide or a pharmaceutical salt thereof as defined in any one of the claims 1-12 or a fusion protein as defined in claim 13 or a pharmaceutical composition as defined in claim 14 for use in therapy.

16. A peptide or a pharmaceutical salt thereof as defined in any one of the claims 1-12 or a fusion protein as defined in claim 13 or the pharmaceutical composition as defined in claim 14 for use in the treatment or prevention of a disease caused by a dysregulation of the VEGF expression level.

17. A peptide or a pharmaceutical salt thereof as defined in any one of the claims 1-12 or a fusion protein as defined in claim 13 or the pharmaceutical composition as defined in claim 14 for use in the treatment or prevention of a disease caused by a dysregulation of the angiogenesis based on the dysregulation of the VEGF expression level.
